(19) **Europäisches Patentamt**
**European Patent Office**
**Office européen des brevets**

(11) **EP 3 066 214 B1**

(12) **EUROPEAN PATENT SPECIFICATION**

(45) Date of publication and mention
of the grant of the patent:
**02.10.2019 Bulletin 2019/40**

(51) Int Cl.:
***C12Q 1/6886*** (2018.01)

(21) Application number: **14803017.4**

(22) Date of filing: **06.11.2014**

(86) International application number:
**PCT/US2014/064378**

(87) International publication number:
**WO 2015/069920 (14.05.2015 Gazette 2015/19)**

(54) **METHODS AND KITS FOR DETECTING A PROSTATE CARCINOMA AND PREDICTING DISEASE OUTCOMES FOR PROSTATE CANCERS**

VERFAHREN UND KITS ZUM NACHWEIS EINES PROSTATAKARZINOMS UND VORHERSAGE VON KRANKHEITSERGEBNISSEN FÜR PROSTATAKREBS

MÉTHODES ET KITS DE DÉTECTION D'UN CANCER DE LA PROSTATE ET DE PRÉDICTION DE L'ÉVOLUTION DES CANCERS DE LA PROSTATE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **07.11.2013 US 201361962465 P**

(43) Date of publication of application:
**14.09.2016 Bulletin 2016/37**

(73) Proprietor: **Cellay, Inc.**
**Cambridge, Massachusetts 02139 (US)**

(72) Inventor: **AURICH-COSTA, Joan**
**Cambridge, MA 02139 (US)**

(74) Representative: **Potter Clarkson**
**The Belgrave Centre**
**Talbot Street**
**Nottingham NG1 5GG (GB)**

(56) References cited:
**WO-A2-2006/089163        US-A- 5 856 097**
**US-A1- 2009 123 054        US-A1- 2012 220 672**
**US-A1- 2012 237 928**

• **JOSEE J KONIG ET AL: "Gain and loss of chromosomes 1, 7, 8, 10, 18, and Y in 46 prostate cancers", HUMAN PATHOLOGY, vol. 27, no. 7, 1 July 1996 (1996-07-01), pages 720-727, XP055145866, ISSN: 0046-8177, DOI: 10.1016/S0046-8177(96)90404-9**

• **ULF S. R. BERGERHEIM ET AL: "Deletion mapping of chromosomes 8, 10, and 16 in human prostatic carcinoma", GENES, CHROMOSOMES AND CANCER, vol. 3, no. 3, 1 May 1991 (1991-05-01), pages 215-220, XP055163508, ISSN: 1045-2257, DOI: 10.1002/gcc.2870030308**

• **IOANNOU D ET AL: "Twenty-four chromosome FISH in human IVF embryos reveals patterns of post-zygotic chromosome segregation and nuclear organisation", CHROMOSOME RESEARCH, KLUWER ACADEMIC PUBLISHERS, DO, vol. 20, no. 4, 29 June 2012 (2012-06-29), pages 447-460, XP035081919, ISSN: 1573-6849, DOI: 10.1007/S10577-012-9294-Z**

• **HAFRON JASON ET AL: "MP69-20 HIGH PREDICTION RATE OF PROSTATE CANCER WITH MINIMALLY INVASIVE OLIGOFISH TEST", JOURNAL OF UROLOGY, vol. 191, no. 4, 28 March 2014 (2014-03-28), XP028602915, ISSN: 0022-5347, DOI: 10.1016/J.JURO.2014.02.2239**

• **ETIM ANN ET AL: "ChromSorter PC: A database of chromosomal regions associated with human prostate cancer", BMC GENOMICS, BIOMED CENTRAL LTD, LONDON, UK, vol. 5, no. 1, 28 April 2004 (2004-04-28), page 27, XP021002107, ISSN: 1471-2164, DOI: 10.1186/1471-2164-5-27**

**(Cont. next page)**

- KOCH J ET AL: "CONSTRUCTION OF A PANEL OF CHROMOSOME-SPECIFIC OLIGONUCLEOTIDE PROBES (PRINS-PRIMERS) USEFUL FOR THE IDENTIFICATION OF INDIVIDUAL HUMAN CHROMOSOMES IN SITU", CYTOGENETICS AND CELL GENETICS, BASEL, CH, vol. 71, no. 2, 1 January 1995 (1995-01-01), pages 142-147, XP008013218, ISSN: 0301-0171
- Michael R Speicher ET AL: "KARYOTYPING HUMAN CHROMOSOMES BY COMBINATORIAL MULTI-FLUOR FISH", NATURE GENETICS, 12 April 1996 (1996-04-12), pages 368-375, XP055376911, DOI: 10.1038/ng0496-368 Retrieved from the Internet: URL:http://www.nature.com/ng/journal/v12/n 4/pdf/ng0496-368.pdf [retrieved on 2017-05-30]
- Kathleen A Cooney ET AL: "Advances in Brief Identification and Characterization of Proximal 6q Deletions in Prostate Cancer'", CANCERRESEARCH, 15 September 1996 (1996-09-15), pages 4150-4153, XP055376919, Retrieved from the Internet: URL:http://cancerres.aacrjournals.org/cont ent/canres/56/18/4150.full.pdf [retrieved on 2017-05-30]
- P. De Mas ET AL: "Increased aneuploidy in spermatozoa from testicular tumour patients after chemotherapy with cisplatin, etoposide and bleomycin", HUMAN REPRODUCTION UPDATE, vol. 16, no. 6, 1 June 2001 (2001-06-01), pages 1204-1208, XP055458011, ISSN: 1355-4786, DOI: 10.1093/humrep/16.6.1204

**Description**

BACKGROUND OF THE INVENTION

Prostate cancer is the most frequently diagnosed cancer in men aside from skin cancer and is the second-leading cause of cancer death in men, with an estimated 29,720 deaths in 2013 (Cancer Facts & Figures 2013, American Cancer Society, pages 19-20). Since early detection and treatment of prostate cancer can enhance patient survival, accurate and reliable methods of detecting this cancer at early (*i.e.*, local or regional) stages are needed.

Current prostate cancer screening methods typically involve testing of prostate-specific antigen (PSA) levels and a digital rectal exam (DRE). Trans-rectal prostate biopsies are then used to detect prostate cancer in patients considered to be at risk. However, PSA testing has a high false positive rate and low clinical specificity, while trans-rectal prostate biopsies have a high false negative rate, do not sample the entire prostate, are highly invasive and painful for most patients and can result in morbidity and mortality. Some publications disclose the use of Fluorescent in Situ Hybridisation (FISH) for detecting prostate cancer like Konig et al., 1996, Human Pathology, pages 720-727 and Bergerheim et al., 1991, Genes Chromosomes and Cancer, pages 215-220. In Ann et al., 2004, BMC Genomics, page 27, a database of chromosomal regions associated with human prostate cancer is disclosed.

Accordingly, there is an urgent need for more accurate and less invasive methods and reagents for detecting prostate cancer.

SUMMARY OF THE INVENTION

The present invention provides a method of diagnosing whether a human subject has a prostate carcinoma, comprising:

a) providing a sample containing prostate cells from the subject;
b) hybridizing a set of at least four chromosome-specific probes to the prostate cells, wherein each chromosome-specific probe has a detectable label and a nucleotide sequence that is complementary to a repetitive genomic sequence of a specific human chromosome, and is specific for a different human chromosome;
wherein the set of chromosome-specific probes includes a chromosome-specific probe for human chromosome 6, a chromosome-specific probe for human chromosome 8, a chromosome-specific probe for human chromosome 10, and a chromosome-specific probe for human chromosome Y; or,
wherein the set of chromosome-specific probes includes a chromosome-specific probe for human chromosome 7, a chromosome-specific probe for human chromosome 16, a chromosome-specific probe for human chromosome 18, and a chromosome-specific probe for human chromosome 20;
c) removing unhybridized probes;
d) detecting the labels on chromosome-specific probes that have hybridized to the prostate cells; and
e) determining whether the prostate cells include prostate cells that are polysomic for at least four chromosomes selected from the group consisting of human chromosome 6, human chromosome 8, human chromosome 10, and human chromosome Y; or human chromosome 7, human chromosome 16, human chromosome 18, and human chromosome 20, wherein the presence of prostate cells that are polysomic for human chromosome 6, human chromosome 8, human chromosome 10, or human chromosome Y; or human chromosome 7, human chromosome 16, human chromosome 18, or human chromosome 20 indicates that the subject has a prostate carcinoma.

Also disclosed herein is, a method of diagnosing whether a human subject has a prostate carcinoma, comprising providing a sample containing prostate cells from the subject; hybridizing a set of at least four chromosome-specific probes, to the prostate cells, wherein each chromosome-specific probe has a detectable label and is specific for a different human chromosome; removing unhybridized probes; detecting the labels on chromosome-specific probes that have hybridized to the prostate cells; and determining whether the prostate cells include polysomic prostate cells, wherein the presence of polysomic prostate cells indicates that the subject has a prostate carcinoma.

The set of chromosome-specific probes may include a chromosome-specific probe for human chromosome 6, a chromosome-specific probe for human chromosome 8, a chromosome-specific probe for human chromosome 10 and chromosome-specific probe for human chromosome Y. The set of chromosome-specific probes may include a chromosome-specific probe for human chromosome 7, a chromosome-specific probe for human chromosome 16, a chromosome-specific probe for human chromosome 18 and a chromosome-specific probe for human chromosome 20. In the context of the present invention, the set of chromosome-specific probes are as defined in the claims.

In another embodiment, the invention relates to a method of diagnosing whether a human subject has a prostate carcinoma, comprising:

a) providing a sample comprising prostate cells from the subject;
b) hybridizing a set of at least four chromosome-specific probes to the prostate cells,

wherein each chromosome-specific probe has a detectable label and a nucleotide sequence that is complementary to a repetitive genomic sequence of a specific human chromosome, and is specific for a different human chromosome selected from the group consisting of human chromosome 6, human chromosome 8, human chromosome 10 and human chromosome Y; or,

wherein the at least four chromosome-specific probes include a chromosome-specific probe for human chromosome 6, a chromosome-specific probe for human chromosome 8, a chromosome-specific probe for human chromosome 10, and a chromosome-specific probe for human chromosome Y;

c) removing unhybridized probes;
d) detecting the labels on chromosome-specific probes that have hybridized to the prostate cells; and
e) determining whether the prostate cells include prostate cells that are polysomic for at least one chromosome selected from the group consisting of chromosome 6, chromosome 8, chromosome 10 and chromosome Y, or a combination thereof,

wherein the presence of prostate cells that are polysomic for at least four chromosomes selected from the group consisting of chromosome 6, chromosome 8, chromosome 10 and chromosome Y, or a combination thereof, indicates that the subject has a prostate carcinoma. Also disclosed herein is a method of diagnosing whether a human subject has a prostate carcinoma, comprising the steps of providing a sample containing prostate cells from the subject; hybridizing a set of at least four, chromosome-specific probes to the prostate cells, wherein each chromosome-specific probe has a detectable label and is specific for a different human chromosome selected from the group consisting of human chromosome 6, human chromosome 8, human chromosome 10 and human chromosome Y; removing unhybridized probes; detecting the labels on chromosome-specific probes that have hybridized to the prostate cells; and determining whether the prostate cells include prostate cells that are polysomic for at least four chromosomes selected from the group consisting of chromosome 6, chromosome 8, chromosome 10 and chromosome Y, or a combination thereof, wherein the presence of prostate cells that are polysomic for at least four chromosomes selected from the group consisting of chromosome 6, chromosome 8, chromosome 10 and chromosome Y, or a combination thereof, indicates that the subject has a prostate carcinoma.

The set of chromosome-specific probes may include a chromosome-specific probe for human chromosome 6, a chromosome-specific probe for human chromosome 8, a chromosome-specific probe for human chromosome 10 and a chromosome-specific probe for human chromosome Y. In the context of the present invention, the set of chromosome-specific probes are as defined in the claims.

In an additional embodiment, the invention provides a kit for detecting polysomic prostate cancer cells in a sample from a human subject,
wherein the kit consists of a set of four chromosome-specific probes, consisting of a chromosome-specific probe for human chromosome 6, a chromosome-specific probe for human chromosome 8, a chromosome-specific probe for human chromosome 10, and a chromosome- specific probe for human chromosome Y; or,
a chromosome-specific probe for human chromosome 7, a chromosome-specific probe for human chromosome 16, a chromosome- specific probe for human chromosome 18, and a chromosome-specific probe for human chromosome 20; wherein the probes are DNA probes, preferably nucleic acid probes; wherein the nucleic acid probes are single stranded, and wherein each chromosome-specific probe has a detectable label and a nucleotide sequence that is complementary to a repetitive genomic sequence of a specific human chromosome; and optionally,
reagents for performing in situ hybridization. Also disclosed herein is a kit for detecting polysomic prostate cancer cells in a sample from a human subject, comprising at least two, preferably three, more preferably four, chromosome-specific probes, wherein each chromosome-specific probe has a detectable label and is specific for a different human chromosome selected from the group consisting of human chromosome Y, human chromosome 6, human chromosome 7, human chromosome 8, human chromosome 10, human chromosome 13, human chromosome 16, human chromosome 18 and human chromosome 20.

The chromosome-specific probes may include a chromosome-specific probe for human chromosome 6 and a chromosome-specific probe for human chromosome 10. The chromosome-specific probes may include a chromosome-specific probe for human chromosome 6, a chromosome-specific probe for human chromosome 8, and a chromosome-specific probe for human chromosome 10. The chromosome-specific probes may include a chromosome-specific probe for human chromosome 6, a chromosome-specific probe for human chromosome 8, a chromosome-specific probe for human chromosome 10 and chromosome-specific probe for human chromosome Y. The chromosome-specific probes may include a chromosome-specific probe for human chromosome 7, a chromosome-specific probe for human chromosome 16, a chromosome-specific probe for human chromosome 18 and a chromosome-specific probe for human chromosome 20. In the context of the present invention, the set of chromosome-specific probes are as defined in the claims.

In yet another embodiment, the invention provides a kit for detecting prostate cancer cells in a sample from a human subject, consisting of a set of probes consisting of four chromosome-specific, single- stranded DNA oligonucleotide probes, wherein the four chromosome- specific probes each has a different detectable label and a nucleotide sequence that is complementary to a repetitive genomic sequence of a specific human chromosome, and is specific for a different human chromosome selected from the group consisting of human chromosome Y, human chromosome 6, human chromosome 7, human chromosome 8, human chromosome 10, human chromosome 16, human chromosome 18 and human chromosome 20; and optionally, additionally consisting of reagents for performing in situ hybridisation. Also disclosed herein is a kit for detecting polysomic prostate cancer cells in a sample from a human subject, comprising a set of probes consisting of four chromosome-specific, single-stranded DNA oligonucleotide probes, wherein the four chromosome-specific probes each has a detectable label and is specific for a different human chromosome selected from the group consisting of human chromosome Y, human chromosome 6, human chromosome 7, human chromosome 8, human chromosome 10, human chromosome 13, human chromosome 16, human chromosome 18 and human chromosome 20.

In an additional embodiment, the invention provides a method of diagnosing whether a human subject has a prostate carcinoma as defined in the claims, comprising applying pressure to the subject's prostate gland, wherein an amount of pressure that is sufficient to release prostate cells into the subject's urethra is applied to the prostate gland; obtaining a urine sample from the subject. In a disclosure, the method further comprises hybridizing a set of at least four chromosome-specific, single-stranded DNA oligonucleotide probes to the prostate cells, wherein each chromosome-specific probe has a detectable label and is specific for a different human chromosome; removing unhybridized probes; detecting the labels on chromosome-specific probes that have hybridized to the prostate cells; and determining whether the prostate cells include polysomic prostate cells, wherein the presence of polysomic prostate cells indicates that the subject has a prostate carcinoma. In a particular disclosure, each of the chromosome-specific probes is specific for a different human chromosome selected from the group consisting of human chromosome Y, human chromosome 6, human chromosome 7, human chromosome 8, human chromosome 10, human chromosome 13, human chromosome 16, human chromosome 18 and human chromosome 20.

In a further embodiment, the invention relates to a method of diagnosing whether a human subject has a prostate carcinoma, as defined in the claims, wherein the combination of probes results in an area under the curve (AUC) of at least 0.80 for a receiver operating characteristic (ROC) curve that is produced when the combination is used to detect polysomic prostate cancer cells. The presence of polysomic prostate cells that are recognized by one or more of the chromosome-specific probes in the combination indicates that the subject has a prostate carcinoma.

In yet another embodiment, the invention relates to a kit, as defined in the claims, wherein the combination of probes results in an area under the curve (AUC) of at least 0.80 for a receiver operating characteristic (ROC) curve that is produced when the combination is used to detect polysomic prostate cancer cells.

In various embodiments, the methods and kits of the present invention provide diagnostic and prognostic tests that are more reliable, more sensitive and less invasive than currently available tests.

BRIEF DESCRIPTION OF THE DRAWINGS

The patent or application file contains at least one drawing executed in color. Copies of this patent or patent application publication with color drawing(s) will be provided by the Office upon request and payment of the necessary fee.

FIG. 1A is an image of a reverse DAPI-stained, fully-karyotyped metaphase showing that chromosome-specific probes for chromosomes 6, 8, 10 and Y (top) and chromosomes 7, 16, 18 and 20 (bottom) hybridize to the correct chromosomes.

FIGS. 1B-1D are fluorescent micrograph images showing human prostatic cells that were hybridized to a panel of four fluorescently-labeled chromosome-specific probes for either chromosomes 7, 16, 18 and 20 (FIG. 1B) or chromosomes 6, 8, 10 and Y (FIGS. 1C, 1D). FIG. 1B shows prostatic cells from a healthy patient. FIG. 1C shows a prostate cell that is missing a Y chromosome (green). FIG. 1D shows prostate cells that are trisomic or tetrasomic for chromosome 8 (blue), indicated by arrows.

FIG.2 is a graph depicting a comparison of the average percentage of cells with chromosomal gains in benign lesions and prostate cancer lesions.

FIG. 3 is a graph depicting a comparison of the average percentage of cells with chromosomal gains in benign lesions, low grade prostate cancer lesions and high grade prostate cancer lesions.

FIG. 4 is a graph showing a receiver operating characteristic (ROC) curve for the differentiation of benign from prostate cancer samples for a specific combination of 8 chromosomes (human chromosome Y, human chromosome 6, human chromosome 7, human chromosome 8, human chromosome 10, human chromosome 16, human chromosome 18 and human chromosome 20).

FIG. 5 is a graph showing an ROC curve for the differentiation of benign from prostate cancer when testing a specific combination of four chromosomes (human chromosome Y, human chromosome 6, human chromosome 8 and human chromosome 10).

FIG. 6 is a graph showing an ROC curve and area under the curve when a combination human chromosome Y, human chromosome 6, human chromosome 7, human chromosome 8, human chromosome 10, human chromosome 16, human chromosome 18 and human chromosome 20 is used to differentiate between low grade and high grade prostate cancer lesions.

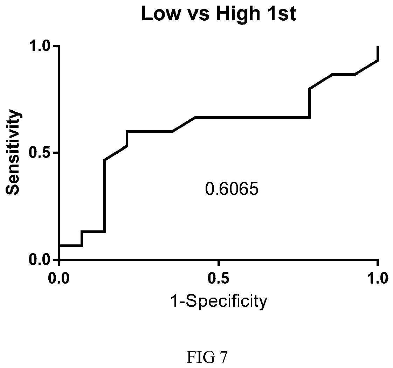

FIG. 7 is a graph showing an ROC curve and area under the curve when a combination of human chromosome Y, human chromosome 6, human chromosome 8 and human chromosome 10 is used for differentiating between low grade and high grade tumor.

FIG. 8 is a graph plotting clinical specificity and sensitivity for different cut-off values for human chromosome Y, human chromosome 6, human chromosome 8 and human chromosome 10.

FIG. 9 is a graph showing the accuracy of individual, and combinations of, chromosome-specific probes for distinguishing benign from cancerous samples based on 64 patient samples.

FIG. 10 is a graph showing the specificity of individual, and combinations of, chromosome-specific probes for distinguishing benign from cancerous samples based on 64 patient samples.

FIG. 11 is a graph showing the sensitivity of individual, and combinations of, chromosome-specific probes for distinguishing benign from cancerous samples based on 64 patient samples.

FIG. 12 is a graph showing the positive prediction value (PPV) of individual, and combinations of, chromosome-specific probes for distinguishing benign from cancerous samples based on 64 patient samples.

FIG. 13 is a graph showing the negative prediction value (NPV) of individual, and combinations of, chromosome-specific probes for distinguishing benign from cancerous samples based on 64 patient samples.

DETAILED DESCRIPTION OF THE INVENTION

Definitions

Unless defined otherwise, all technical and scientific terms used herein have the same meaning as commonly understood by one of ordinary skill in the art to which this invention pertains.

The term "nucleotide" refers to naturally occurring ribonucleotide or deoxyribonucleotide monomers, as well as non-naturally occurring derivatives and analogs thereof. Accordingly, nucleotides can include, for example, nucleotides comprising naturally occurring bases (*e.g.*, A, G, C, or T) and nucleotides comprising modified bases (*e.g.*, 7-deazaguanosine, or inosine).

The term "sequence," in reference to a nucleic acid, refers to a contiguous series of nucleotides that are joined by covalent bonds (*e.g.*, phosphodiester bonds).

The term "nucleic acid" refers to a polymer having multiple nucleotide monomers. A nucleic acid can be single- or double-stranded, and can be DNA (*e.g.*, cDNA or genomic DNA), RNA, or hybrid polymers (*e.g.*, DNA/RNA). Nucleic acids can be chemically or biochemically modified and/or can contain non-natural or derivatized nucleotide bases. Nucleic acid modifications include, for example, methylation, substitution of one or more of the naturally occurring nucleotides with an analog, internucleotide modifications such as uncharged linkages (*e.g.*, methyl phosphonates, phosphotriesters, phosphoamidates, carbamates, and the like), charged linkages (*e.g.*, phosphorothioates, phosphorodithioates, and the like), pendent moieties (*e.g.*, polypeptides), intercalators (*e.g.*, acridine, psoralen, and the like), chelators, alkylators, and modified linkages (*e.g.*, alpha anomeric nucleic acids, and the like). Nucleic acids also include synthetic molecules that mimic nucleic acids in their ability to bind to a designated sequence via hydrogen bonding and other chemical interactions. Typically, the nucleotide monomers are linked via phosphodiester bonds, although synthetic forms of nucleic acids can comprise other linkages (*e.g.*, peptide nucleic acids (also referred to herein as "PNAs"), such as described in Nielsen et al., Science 254, 1497-1500, 1991). Nucleic acids can also include, for example, conformationally restricted nucleic acids (*e.g.*, "locked nucleic acids" or "LNAs," such as described in Nielsen et al., J. Biomol. Struct. Dyn. 17:175-91, 1999), morpholinos, glycol nucleic acids (GNA) and threose nucleic acids (TNA). "Nucleic acid" does not refer to any particular length of polymer and can, therefore, be of substantially any length, typically from about six (6) nucleotides to about $10^9$ nucleotides or larger. In the case of a double-stranded polymer, "nucleic acid" can refer to either or both strands.

The term "target nucleic acid" refers to a nucleic acid whose presence or absence in a sample is desired to be detected. The term "target sequence" refers to a nucleotide sequence in a target nucleic acid that is capable of forming a hydrogen-bonded duplex with a complementary sequence (*e.g.*, a substantially complementary sequence) on an oligonucleotide probe.

As used herein, "complementary" refers to sequence complementarity between two different nucleic acid strands or between two regions of the same nucleic acid strand. A first region of a nucleic acid is complementary to a second region of the same or a different nucleic acid if, when the two regions are arranged in an anti-parallel fashion, at least one nucleotide residue of the first region is capable of base pairing (*i.e.*, hydrogen bonding) with a residue of the second region, thus forming a hydrogen-bonded duplex.

The term "substantially complementary" refers to two nucleic acid strands (*e.g.*, a strand of a target nucleic acid and a complementary single-stranded oligonucleotide probe) that are capable of base pairing with one another to form a stable hydrogen-bonded duplex under stringent hybridization conditions, including the isothermal hybridization conditions described herein. In general, "substantially complementary" refers to two nucleic acids having at least 70%, for example, about 75%, 80%, 85%, 90%, 95%, 97%, 98%, 99% or 100% complementarity.

The term "probe" refers to a molecule that includes a target-binding region that is substantially complementary to a target sequence in a target nucleic acid and, thus, is capable of forming a hydrogen-bonded duplex with the target nucleic acid. Typically, the probe is a single-stranded probe, having one or more detectable labels to permit the detection of the probe following hybridization to its complementary target.

As used herein, "target-binding region" refers to a portion of an oligonucleotide probe that is capable of forming a hydrogen-bonded duplex with a complementary target nucleic acid.

A "linker," in the context of attachment of two molecules (whether monomeric or polymeric), means a molecule (whether monomeric or polymeric) that is interposed between and adjacent to the two molecules being attached. A "linker" can be used to attach, *e.g.*, oligonucleotide probe sequence and a label (*e.g.*, a detectable label). The linker can be a nucleotide linker (*i.e.*, a sequence of the nucleic acid that is between and adjacent to the non-adjacent sequences) or a non-nucleotide linker.

The term "hybrid" refers to a double-stranded nucleic acid molecule formed by hydrogen bonding between complementary nucleotides.

The term "stringency" refers to hybridization conditions that affect the stability of hybrids, *e.g.*, temperature, salt concentration, pH, formamide concentration, and the like. These conditions are empirically optimized to maximize specific binding, and minimize nonspecific binding, of a probe to a target nucleic acid.

The term "fluorophore" refers to a chemical group having fluorescence properties.

Methods of Diagnosing a Prostate Cancer

In one embodiment, the present invention provides a method of diagnosing (*e.g.*, detecting) whether a human subject has a prostate carcinoma as defined in the claims. The presence of polysomic prostate cells is indicative of a prostate carcinoma in the subject. As used herein, "polysomic prostate cells" refers to prostate cells (*e.g.*, prostate epithelial cells) that have one or more extra copies (*i.e.*, three or more copies of a human autosome; two or more copies of a human Y chromosome) of one or more human chromosomes (*e.g.*, human chromosome Y, human chromosome 6, human chromosome 7, human chromosome 8, human chromosome 10, human chromosome 13, human chromosome 16, human chromosome 18, human chromosome 20).

A description of steps that can be included in the methods described herein are set forth below.

Obtaining a urine sample containing prostate cells

Urine samples that are suitable for the methods described herein contain a sufficient number of prostate cells (*e.g.*, epithelial cells of prostatic origin), also referred to as prostatic cells, to allow for the detection of polysomic prostate cells. Typically, a urine sample containing voided urine will not be suitable for use in the claimed methods unless, prior to obtaining the urine sample, the subject is treated in a manner that causes prostatic fluid containing prostate cells (*e.g.*, prostate epithelial cells) to be released from the subject's prostate gland into the urethra. Once in the urethra, the prostatic fluid will mix with urine such that subsequently voided urine from the subject will contain a sufficient number of prostate cells for analysis by the methods described herein.

In one embodiment, pressure is applied to the subject's prostate gland to release prostatic fluid into the subject's urethra. The pressure can be applied manually by a skilled medical professional (*e.g.*, by a physician during a rectal examination of the subject). Typically, the examining medical professional applies pressure to the subject's prostate gland with his or her finger, although a suitable instrument, probe or device can be used as an alternative means of applying pressure to the prostate gland. A skilled medical professional (*e.g.*, physician) can readily determine how much pressure should be applied to the prostate gland to release prostatic fluid into the urethra.

An exemplary procedure for applying pressure to a subject's prostate gland and releasing prostatic fluid into the urethra is described in the Materials and Methods of the Example disclosed herein. Briefly, a skilled medical professional can perform a digital rectal examination (DRE) that includes stroking the lobes of the subject's prostate gland through the rectum with a depression of approximately 1 centimeter. The lobes are stroked from base to apex and from the lateral to the median line. Typically, the lobes of the prostate gland will be stroked multiple times (*e.g.*, three times).

Preferably, voided urine is obtained from the subject within minutes of the application of pressure to the subject's prostate gland. In a particular embodiment, a voided urine sample is obtained from the subject immediately after the procedure for applying pressure to the subject's prostate gland has been completed.

Hybridizing a set of at least two chromosome-specific probes to the prostate cells

The methods described herein also include the step of hybridizing a set of at least two chromosome-specific probes to the prostate cells. Preferably, each chromosome-specific probe in the set has a detectable label (*e.g.*, a direct detectable label, an indirect detectable label) and is specific for a different human chromosome.

As used herein, the term "probe" refers to a molecule (*e.g.*, a nucleic acid) that includes a target-binding region that is substantially complementary to a target sequence in a target nucleic acid and, thus, is capable of forming a hydrogen-bonded duplex with the target nucleic acid.

Probes that are useful in the methods and kits described herein are chromosome-specific probes (*e.g.*, chromosome-specific nucleic acid probes). A "chromosome-specific probe," as used herein, refers to a probe that specifically binds (*e.g.*, hybridizes) to a particular human chromosome, but does not bind to other human chromosomes, under standard hybridization conditions. The chromosome-specific probes can comprise a sequence that is complementary (*e.g.*, perfectly complementary, at least 90% complementary) to a unique (*e.g.*, non-repetitive) sequence, or a repeat sequence (*e.g.*, a repetitive genomic sequence) of a specific human chromosome (*i.e.*, human chromosome 1, 2, 3, 4, 5, 6, 7, 8, 9, 10, 11, 12, 13, 14, 15, 16, 17, 18, 19, 20, 21, 22, the human X chromosome or the human Y chromosome). "Repeat sequence" or "repetitive sequence" refers to noncoding tandemly repeated nucleotide sequences in the human genome including, e.g., repeat sequences from the alpha satellite, satellite 1, satellite 2, satellite 3, the beta satellite, the gamma satellite and telomeres. Repeat sequences are known in the art and are described in *e.g.*, (Allshire et al., Nucleic Acids Res 17(12): 4611-27 (1989); Cho et al., Nucleic Acids Res 19(6): 1179-82 (1991); Fowler et al., Nucleic Acids Res 15(9): 3929 (1987); Haaf et al., Cell 70(4): 681-96 (1992); Lee et al., Chromosoma 109(6): 381-9 (2000); Maeda and Smithies, Annu Rev Genet 20: 81-108 (1986); Meyne and Goodwin, Chromosoma 103(2): 99-103 (1994); Miklos (1985). Localized highly repetitive DNA sequences in vertebrate genomes. Molecular evolutionary genetics. I. J. R. Macintyre. NY, Plenum Publishing Corp.: 241-321 (1985); Tagarro et al., Hum Genet 93(2): 125-8 (1994); Waye and Willard, PNAS USA 86(16): 6250-4 (1989); and Willard and Waye, J Mol Evol 25(3): 207-14 (1987). The repeat sequences are located at, *e.g.*, the centromeric, pericentromeric, heterochromatic, and telomeric regions of chromosomes.

Suitable repeat sequences include, but are not limited to, a centromeric repeat sequence, a pericentromeric repeat sequence, a heterochromatin repeat sequence, a telomeric repeat sequence, an alpha satellite repeat sequence, a beta satellite repeat sequence, a gamma satellite repeat sequence, and a satellite 1, 2, or 3 repeat sequence. In a particular embodiment, the chromosome-specific probes are complementary to a centromeric repeat sequence of a specific human chromosome. Such probes are referred to herein as centromeric probes.

Although generally desirable, a probe is not required to have 100% complementarity to a chromosome-specific sequence of a human chromosome. For example, in some embodiments, probes useful in the methods of the invention can comprise a nucleotide sequence that is about 70%, about 80%, about 90%, about 95% or about 99%, complementary to a specific nucleotide sequence of a human chromosome.

In some embodiments, the chromosome-specific sequence in the probe is less than 84% identical to a consensus repeat sequence (e.g., an alpha satellite repeat sequence, a beta satellite repeat sequence, a gamma satellite repeat sequence, a telomeric repeat sequence, or a satellite 1, 2, or 3 repeat sequence) of all human chromosomes. In certain embodiments, the chromosome-specific sequence is less than 85%, less than 84%, less than 80%, less than 78%, less than 75%, less than 73%, or less than 70% identical to the consensus repeat sequence. Preferably, the synthetic oligonucleotide is less than 84% identical to all other contiguous nucleic acid sequences within the human genome. Consensus repeat sequences are described in, *e.g.* Willard and Waye, J Mol Evol 25(3): 207-14 (1987) and Tagarro et al., Hum Genet 93(2): 125-8 (1994). Vissel and Choo, Nucleic Acids Res. 15(16): 6751-6752 (1987), Cho et al., Nucleic Acids Res 19(6): 1179-82 (1991).

The set of probes employed in the methods and kits described herein may include at least two chromosome-specific probes that are specific for different human chromosomes selected from the group consisting of human chromosome Y, human chromosome 6, human chromosome 7, human chromosome 8, human chromosome 10, human chromosome 13, human chromosome 16, human chromosome 18 and human chromosome 20.

The sets of chromosome-specific probes may consist of two chromosome-specific probes that are specific for different human chromosomes selected from the group consisting of human chromosome Y, human chromosome 6, human chromosome 7, human chromosome 8, human chromosome 10, human chromosome 13, human chromosome 16, human chromosome 18 and human chromosome 20. The sets of chromosome-specific probes may include more than two chromosome-specific probes, for example, three, four, five, six, seven or eight chromosome-specific probes that are specific for different human chromosomes selected from the group consisting of human chromosome Y, human chromosome 6, human chromosome 7, human chromosome 8, human chromosome 10, human chromosome 13, human chromosome 16, human chromosome 18 and human chromosome 20. Preferably, sets of chromosome-specific probes consist of two, preferably three, more preferably four chromosome-specific probes. In the context of the present invention, the set of chromosome-specific probes are as defined in the claims.

Sets of chromosome-specific probes that are particularly suitable for use in the methods described herein preferably include a chromosome-specific probe for human chromosome 6 and a chromosome-specific probe for human chromosome 10.

A set of chromosome-specific probes may include a chromosome-specific probe for human chromosome 6, a chromosome-specific probe for human chromosome 8, and a chromosome-specific probe for human chromosome 10. In the context of the present invention, the set of chromosome-specific probes are as defined in the claims.

A set of chromosome-specific probes may include a chromosome-specific probe for human chromosome 6, a chromosome-specific probe for human chromosome 8, a chromosome-specific probe for human chromosome 10 and chromosome-specific probe for human chromosome Y.

A set of chromosome-specific probes may include a chromosome-specific probe for human chromosome 7 and a chromosome-specific probe for human chromosome 20.

A set of chromosome-specific probes may include a chromosome-specific probe for human chromosome 7, a chromosome-specific probe for human chromosome 16, a chromosome-specific probe for human chromosome 18 and a chromosome-specific probe for human chromosome 20.

A set of chromosome-specific probes may include a chromosome-specific probe for human chromosome 7, a chromosome-specific probe for human chromosome 16, a chromosome-specific probe for human chromosome 18 and a chromosome-specific probe for human chromosome Y.

Typically, the probes employed in the methods and kits described herein are nucleic acid probes. Suitable nucleic acid probes include, but are not limited to, DNA probes, RNA probes, peptide nucleic acid (PNA) probes, locked nucleic acid (LNA) probes, morpholino probes, glycol nucleic acid (GNA) probes and threose nucleic acid (TNA) probes, as well as combinations thereof. Such probes can be chemically or biochemically modified and/or can contain non-natural or derivatized nucleotide bases. For example, a probe can contain modified nucleotides having modified bases (*e.g.*, 5-methyl cytosine) and/or modified sugar groups (*e.g.*, 2'O-methyl ribosyl, 2'O-methoxyethyl ribosyl, 2'-fluoro ribosyl, 2'-amino ribosyl). Although linear probes are preferred, useful probes can be circular or branched and/or include domains capable of forming stable secondary structures (*e.g.*, stem- and- loop and loop-stem-loop hairpin structures). Preferably, the probes are DNA probes.

Although single-stranded nucleic acid probes are preferred, suitable probes for the methods described herein can be double stranded (*e.g.*, dsDNA) or single stranded (*e.g.*, ssDNA). Preferably, the probes are single-stranded DNA probes.

Methods of producing probes useful in the methods of the invention are well known in the art and include, for example, biochemical, recombinant, synthetic (*e.g.*, chemical synthesis) and semi-synthetic methods. In one embodiment, the oligonucleotide probes employed in the methods of the invention are produced by chemical synthesis. A synthetic oligonucleotide probe can be produced using known methods for nucleic acid synthesis (see, *e.g.*, Glick and Pasternak, Molecular Biotechnology: Principles and Applications of Recombinant DNA (ASM Press 1998)). For example, solution or solid-phase techniques can be used. Synthesis procedures are typically automated and can include, for example, phosphoramidite, phosphite triester, H-phosphate, or phosphotriester methods.

In a particular embodiment, the probes are oligonucleotide probes (*e.g.*, single-stranded DNA oligonucleotide probes). Typical oligonucleotide probes are linear and range in size from about 20 to about 100 nucleotides, preferably, about 25 to about 50 nucleotides. In a particular embodiment, the oligonucleotide probes in a kit are about 30 nucleotides in length. Typically, the oligonucleotide probes target repetitive genomic DNA sequences (*e.g.*, centromeric repetitive DNA sequences). Preferably, the probes are centromeric probes that are complementary to one or more repeat sequences at or near a chromosome's centromere.

In another embodiment, probes that are prepared from genomic fragments can be used. Such genomic fragments can be obtained by a variety of procedures that are well known in the art, including, but not limited to, amplification of genomic DNA (*e.g.*, by polymerase chain reaction (PCR), such as long-range PCR), nuclease digestion of cloned DNA fragments present in, for example, plasmids, cosmids, artificial chromosomes (*e.g.*, bacterial artificial chromosomes (BACs), yeast artificial chromosomes (YACs), PI-derived artificial chromosomes and mammalian artificial chromosomes) and phages, microdissection of chromosomes, and sorting of whole chromosomes by flow cytometry. Preferably, the probes prepared from genomic fragments target unique, non-repetitive genomic sequences.

Preferably, the probes have one or more detectable labels. The term "detectable label," as used herein, refers to a moiety that indicates the presence of a corresponding molecule (*e.g.*, probe) to which it is bound. Labels suitable for use according to the present invention are known in the art and generally include any molecule that, by its chemical nature, and whether by direct or indirect means, provides an identifiable signal allowing detection of the probe. Thus, for example, probes can be labeled in a conventional manner, such as with specific reporter molecules, fluorophores, radioactive materials, or enzymes (*e.g.*, peroxidases, phosphatases). In a particular embodiment, the probes employed in the methods of the invention include one or more fluorophores as detectable labels.

In one embodiment, each chromosome-specific probe can have a detectable label that differs from the detectable labels on the other chromosome-specific probes. In another embodiment, each chromosome-specific probe can have a detectable label that is the same as the detectable labels on the other chromosome-specific probes.

Detectable labels suitable for attachment to probes can be indirect labels or direct labels. An "indirect label" refers to a moiety, or ligand, that is detected using a labeled secondary agent, or ligand-binding partner, that specifically binds to the indirect label. Exemplary indirect labels include, *e.g.*, haptens, biotin, or other specifically bindable ligands. For indirect labels, the ligand-binding partner typically has a direct label, or, alternatively, is also labeled indirectly. Examples of indirect labels that are haptens include dinitrophenol (DNP), digoxigenin, biotin, and various fluorophores or dyes (*e.g.*, fluorescein, DY490, DY590, Alexa 405/Cascade blue, Alexa 488, Bodiby FL, Dansyl, Oregon Green, Lucifer Yellow, Tetramethylrhodamine/ Rhodamine Red, and Texas Red). As an indirect label, a hapten is typically detected using an anti-hapten antibody as the ligand-binding partner. However, a hapten can also be detected using an alternative ligand-binding partner (*e.g.*, in the case of biotin, anti-biotin antibodies or streptavidin, for example, can be used as the ligand-binding partner). Further, in certain embodiments, a hapten can also be detected directly (*e.g.*, in the case of fluorescein, an anti-fluorescein antibody or direct detection of fluorescence can be used).

Preferably, the probes have a direct label. As used herein, a "direct label" refers to a moiety that is detectable in the absence of a ligand-binding partner interaction. Exemplary "direct labels" include, but are not limited to, fluorophores (*e.g.*, fluorescein, rhodamine, Texas Red, phycoerythrin, Cy3, Cy5, DY fluors (Dyomics GmbH, Jena, Germany) Alexa 532, Alexa 546, Alexa 568, or Alexa 594), which are also referred to as fluors or fluorescent labels. Other direct labels can include, for example, radionuclides (*e.g.*, 3H, 35S, 32P, 125I, and 14C), enzymes such as, *e.g.*, alkaline phosphatase, horseradish peroxidase, or β-galactosidase, chromophores (*e.g.*, phycobiliproteins), luminescers (*e.g.*, chemiluminescers and bioluminescers), and lanthanide chelates (*e.g.*, complexes of Eu3+ or Tb3+). Preferably, the direct label is a fluorescent label. In the case of fluorescent labels, fluorophores are not to be limited to single species organic molecules, but include inorganic molecules, multi-molecular mixtures of organic and/or inorganic molecules, crystals, heteropolymers, and the like. For example, CdSe-CdS core-shell nanocrystals enclosed in a silica shell can be easily derivatized for coupling to a biological molecule (Bruchez et al., Science, 281:2013-2016, 1998). Similarly, highly fluorescent quantum dots (zinc sulfide-capped cadmium selenide) have been covalently coupled to biomolecules for use in ultrasensitive biological detection (Warren and Nie, Science, 281: 2016-2018, 1998).

Probe labeling can be performed, *e.g.*, during synthesis or, alternatively, post-synthetically, for example, using 5'-end labeling, which involves the enzymatic addition of a labeled nucleotide to the 5'-end of the probe using a terminal transferase. A single labeled nucleotide can be added by using a "chain terminating" nucleotide. Alternatively, non-terminating nucleotides can be used, resulting in multiple nucleotides being added to form a "tail." For synthesis labeling, labeled nucleotides (*e.g.*, phosphoramidite nucleotides) can be incorporated into the probe during chemical synthesis. Labels can be added to the 5', 3', or both ends of the probe (see, *e.g.*, U.S. Patent No. 5,082,830), or at base positions internal to the ODN.

Suitable methods for labeling probes (*e.g.*, nucleic acid probes) with one or more detectable labels (*e.g.*, direct labels, indirect labels) are well known in the art and include, for example, nick translation, random priming and PCR-based labeling techniques.

Other methods for labeling nucleic acids utilize platinum-based labeling. Such methods include the Universal Linkage System (ULS, Kreatech Biotechnology B.V., Amsterdam, Netherlands). Platinum based labeling methods and their applications are described in, for example, U.S. Patent Nos. 5,580,990, 5,714,327, and 6,825,330; International Patent Publication Nos. WO 92/01699, WO 96/35696, and WO 98/15546; Hernandez-Santoset et al., Anal. Chem. 77:2868-2874, 2005; Raap et al., BioTechniques 37:1-6, 2004; Heetebrij et al., ChemBioChem 4:573-583, 2003; Van de Rijke et al., Analytical Biochemistry 321:71-78, 2003; Gupta et al., Nucleic Acids Research 31:e13, 2003; Van Gijlswijk et al., Clinical Chemistry 48:1352-1359, 2002; Alers et al., Genes, Chromosomes & Cancer 25:301-305, 1999; Wiegant et al., Cytogenetics and Cell Genetics 87:7-52, 1999; Jelsma et al., Journal of NIH Research 5:82, 1994; Van Belkum et al., BioTechniques 16:148-153, 1994; and Van Belkum et al., Journal of Virological Methods 45:189-200, 1993.

Labeled nucleotide(s) can also be attached to a probe using a crosslinker or a spacer. Crosslinkers can be homobifunctional or heterobifunctional. Suitable homobifunctional crosslinkers include, *e.g.*, amine reactive crosslinkers with NHS esters at each end (including, *e.g.*, dithiobis(succinimidylproponate) (DSP); 3, 3'-dithiobis(sulfosuccinimidylpropionate) (DTSSP); disuccinimidyl suberate (DSS); Bis(sulfosuccinimidyl)suberate (BS3); Ethylene glycolbis(succinimidylsuccinate) (EGS); Ethylene glycolbis(sulfosuccinimidylsuccinate) (SulfoEGS)); amine reactive crosslinkers with imidoesters at both ends (including, *e.g.*, dimethyl adipimidate (DMA); dimethyl pimelimidate (DMP); dimethyl suberimidate (DMS); dimethyl 3,3'-dithiobispropionimidate (DTBP)); sulfhydryl reactive crosslinkers with dithiopyridyl groups at each end (including, *e.g.*, 1,4-di-[3'-(2'-pyridyldithio)propionamdo]butane (DPDPB)); sulfhydryl reactive crosslinkers with maleimide groups at each end (including, *e.g.*, bismaleimidohexane (BMH)); carboxyl reactive crosslinkers with hydrazide groups at each end (including, *e.g.*, adipic acid dihydrazide and carbonhydrazide); multi-group reactive crosslinkers with epoxide groups at each end (including, *e.g.*, 1,2:3,4-diepoxybutane; 1,2:5,6-diepoxyhexane; Bis(2,3-epoxypropyl)ether; 1,4-(butanediol) diglycidyl ether). Suitable heterobifunctional crosslinkers include crosslinkers with an amine reactive end and a sulfhydryl-reactive end (including, *e.g.*, N-Succinimidyl 3-(2-pyridyldithio)propionate (SPDP); long chain SPDP (SPDP); Sulfo-LC-SPDP; Succinimidyloxycarbonyl-α-methyl-α-(2-pyridydithio)toluene (SMPT); Sulfo-LC-SMPT; Succinimidyl-4-(N-maleimidomehyl)cyclohexane (SMCC); Sulfo-SMCC; Succinimidyl 6-((iodoacetyl)amino)hexanoate (SIAX); Succinimidyl 6-(6-(((4-iodoacetyl)amino)hexanoyl)amino)hexanoate (SIAXX)); crosslinkers with a carbonyl-reactive end and a sulfhydryl reactive end (including, *e.g.*, 4-(4-N-Maleimidophenyl)butyric acid hydrazide (MPBH); 4-(N-Maleimidomethyl)cyclohexane-1-carboxyl-hydrazide hydrochloride (M2C2H); 3-(2-Pyridyldithio)propinyl hydrazide (PDPH)); crosslinkers with an amine-reactive end and a photoreactive end (including, *e.g.*, Sulfosuccinimidyl-2-(p-azidosalicylicylamido)ethyl-1,3'-dithiopropionate (SASD); Sulfosuccinimidyl 2-(7-azido-4-methylcoumarin-3-acetamide)ethyl-1,3'- dithiopropionate (SAED)); crosslinkers with a sulfhydryl-reactive end and a photoreactive end (including, *e.g.*, N-[4-p-Azidosalicylamido)butyl]-3'-(2'pyridyldithio)propionamide (APDP)); crosslinkers with a carbonyl-reactive end and a photoreactive end (including, *e.g.*, 4-(p-Azidosalicylamido)butlyamine (ASBA)). Suitable spacers include, 5' ODN modifications such as dNTP's; and amine-reactive spacers such as amino- or sulfo-phosphoramidites including, *e.g.*, butylphosphoramidites, pentylphosphoramidites, hexylphosphoramidites, heptylphosphoramidites, octylphosphoramidites, nonylphosphoramidites, decylphosphoramidites, undecylphosphoramidites, dodecylphosphoramidites, pentadecylphosphoramidites, octadecylphosphoramidites. Other suitable amine-reactive spacers include *e.g.*, activated polyethylene glycol (PEG) such as (monomethoxy)n glycol, wherein n=3-18 unit repeats. Additional suitable crosslinkers and spacers are set forth in Herman. "Bioconjugate Chemistry". Academic Press. New York, NY. 1996.

Generally, hybridization is performed under conditions (*e.g.*, temperature, incubation time, salt concentration, etc.) sufficient for a probe to hybridize with a complementary target nucleic acid in a sample (*e.g.*, prostate cells). Suitable hybridization buffers and conditions for *in situ* hybridization techniques are generally known in the art. (See, *e.g.*, Sambrook and Russell, supra; Ausubel et al., supra. See also Tijssen, Laboratory Techniques in Biochemistry and Molecular Biology, Vol. 24: Hybridization with Nucleic Acid Probes (Elsevier, NY 1993)).

Hybridization is typically carried out under stringent conditions that allow formation of stable and specific binding of substantially complementary strands of nucleic acid and any washing conditions that remove non-specific binding of the probe. Generally, stringency occurs within a range from about 5° C below the melting temperature ($T_m$) of the probe to about 20° C-25° C below the $T_m$. Stringency can be increased or decreased to specifically detect target nucleic acids having 100% complementarity or to also detect related nucleotide sequences having less than 100% complementarity. In certain methods, very stringent conditions are selected to be equal to the $T_m$ for a particular probe. Factors such as the length and nature (DNA, RNA, base composition) of the sequence, nature of the target (DNA, RNA, base composition, presence in solution or immobilization) and the concentration of the salts and other components (*e.g.*, the presence or absence of formamide, dextran sulfate and/or polyethylene glycol) are considered and the hybridization solution can be varied to generate conditions of either low, medium, or high stringency. Washing conditions typically range from room temperature to 60° C.

For example, standard, high stringency conditions can include, e.g., 2xSSC, 30% formamide, 10% Dextran Sulfate at 37°C for the hybridization, followed by 3 washes at 45°C in 30% formamide 2xSSC, 5 washes in 2xSSC at 45°C and one wash at 45°C in 1xPBD (Na2HPO4 0.1 M, NaH$_2$PO$_4$ 0.6 mM, NaN$_3$ 0,003%, Nonidet P40 0,05%). Other suitable high stringency conditions include 6×NaCl/sodium citrate (SSC) at about 45 °C for a hybridization step, followed by a wash of 2×SSC at 50 °C; or hybridization at 42 °C in 5×SSC, 20 mM NaPO$_4$, pH 6.8, 50% formamide, followed by a wash of 0.2×SSC at 42 °C. Other suitable high stringency conditions include 0.5xSSC, 0.1%SDS at 50°C for 2 minutes These conditions can be varied based on nucleotide base composition and length and circumstances of use, either empirically or based on formulas for determining such variation (*see*, *e.g.*, Sambrook *et al.*, *supra*; Ausubel *et al.*, *supra*). Depending on base composition, source, and concentration of target nucleic acid, other conditions of stringency can be used, including, for example, low stringency conditions (*e.g.*, 4-6×SSC/0.1-0.5% w/v SDS at 37-45 °C for 2-3 hours) or medium stringency conditions (*e.g.*, 1-4×SSC/0.25-0.5% w/v SDS at 45 °C for 2-3 hours).

In general, there is a tradeoff between hybridization specificity (stringency) and signal intensity. In certain embodiments, the hybridized sample can be washed at successively higher stringency solutions and read between each wash. Analysis of the data sets thereby produced reveals a wash stringency above which the hybridization pattern is not appreciably altered and which provides adequate signal for the particular probes of interest.

Certain differences in the conditions used for hybridization and detection can arise from differences in the detectable label used. For example, when biotin-labeled probes are used, polyvinylpyrrolidone, a constituent of Denhardt's solution, is omitted from the hybridization solution. As another simple example, when the probe is labeled with a fluorescent label, the probe is generally protected from bright light. These differences and others are known to those skilled in the use of various detectable labels that can be attached to a probe in accordance with the present invention.

In one embodiment, a hybridization buffer comprising formamide, dextran sulfate and saline sodium citrate (SSC) can be employed in the methods of the invention. Suitable concentrations of formamide in the hybridization buffer include, for example, concentrations in the range of about 20% to about 90% by volume, e.g., about 60%, about 70%,

or about 80% by volume. Suitable concentrations of dextran sulfate in a hybridization buffer include, for example, about 3% to about 20%. Suitable concentrations of SSC in a hybridization buffer include, for example, about 0.1 × to about 4×. The concentration of total salt in the hybridization buffer is preferably in the range of about 0.03M to about 0.09M.

In a particular embodiment, the hybridization step is performed under alkaline conditions. For example, a hybridization buffer containing one or more bases (*e.g.*, NaOH) and having a pH of about 10 to about 13, preferably about 11 to about 12, is employed. Suitable bases for hybridization buffers include, without limitation, potassium hydroxide, barium hydroxide, caesium hydroxide, sodium hydroxide, strontium hydroxide, calcium hydroxide, lithium hydroxide, rubidium hydroxide, magnesium hydroxide, butyl lithium, lithium diisopropylamide, lithium diethylamide, sodium amide, sodium hydride, lithium bis(trimethylsilyl)amide, sodium carbonate and ammonia, or a combination thereof. Preferably, the base is an alkali base. More preferably, the base is sodium hydroxide. Suitable alkaline hybridization buffers that can be employed in the methods of the invention include, for example, about 20-60% formamide, about 5-40% dextran sulfate and about 1-10 mM NaOH, and have a pH in the range of about 10 to about 13. Preferably, the alkaline hybridization buffer contains about 30-50% formamide, about 10% dextran sulfate and about 1-3 mM NaOH, and has a pH in the range of about 11 to about 12. Such conditions are particularly suitable when repetitive sequence probes (*e.g.*, oligonucleotide probes) are employed in the methods described herein. An exemplary hybridization buffer for use in the methods of the invention when unique-sequence probes (*e.g.*, BAC probes) are employed, includes, for example, about 4.2 mM NaOH, about 42% formamide and about 28% dextran sulfate.

Optimal hybridization conditions for a given target sequence and its complementary probe will depend upon several factors such as salt concentration, incubation time, and probe concentration, composition, and length, as will be appreciated by those of ordinary skill in the art. Based on these and other known factors, suitable binding conditions can be readily determined by one of ordinary skill in the art and, if necessary, optimized for use in accordance with the present methods. Typically, hybridization is carried out under stringent conditions that allow specific binding of substantially complementary nucleotide sequences. Stringency can be increased or decreased to specifically detect target nucleic acids having 100% complementarity or to detect related nucleotide sequences having less than 100% complementarity (*e.g.*, about 70% complementarity, about 80% complementarity, about 90% complementarity). Factors such as the length and nature (DNA, RNA, base composition) of the probe sequence, nature of the target nucleotide sequence (DNA, RNA, base composition, presence in solution or immobilization) and the concentration of salts and other components in the hybridization buffer (*e.g.*, the concentration of formamide, dextran sulfate, polyethylene glycol and/or salt) in the hybridization buffer/solution can be varied to generate conditions of either low, medium, or high stringency. These conditions can be varied based on nucleotide base composition and length and circumstances of use, either empirically or based on formulas for determining such variation (see, *e.g.*, Sambrook et al., supra; Ausubel et al., supra).

In one embodiment, the step of hybridizing a set of at least two chromosome-specific probes to the prostate cells is performed at room temperature. As used herein, the terms "room temperature" and "RT" refer to temperatures in the range of about 19 degrees Celsius to about 25 degrees Celsius. For example, hybridization can be performed at a temperature in the range of about 19° C to about 25° C, preferably about 20° C to about 22° C, more preferably about 21° C. Exemplary conditions for performing probe hybridizations at room temperature are described, for example, in U.S. Patent Application Publication Nos. US 2013/0203055 A1.

Removing unhybridized probes

The methods described herein can further include the step of removing unhybridized probes after hybridizing a set of at least two chromosome-specific probes to the sample (*e.g.*, prostate cells). For example, the sample (*e.g.*, prostate cells) can be washed (*e.g.*, in a wash buffer) after the hybridization step. Typically, washes are performed in a solution of appropriate stringency to remove unbound and/or non-specifically bound probes. An appropriate stringency can be determined, for example, by washing the sample in successively higher stringency solutions and reading the signal intensity between each wash. Analysis of the data sets in this manner can reveal a wash stringency above which the hybridization pattern is not appreciably altered and which provides adequate signal for the particular probes of interest. In addition, the number of washes and duration of each wash can be readily determined by one of ordinary skill in the art.

Suitable wash buffers for *in situ* hybridization methods are generally known in the art (See, *e.g.*, Sambrook and Russell, supra; Ausubel et al., supra. See also Tijssen, Laboratory Techniques in Biochemistry and Molecular Biology, Vol. 24: Hybridization with Nucleic Acid Probes (Elsevier, NY 1993)). Wash buffers typically include, for example, one or more salts (*e.g.*, sodium salts, lithium salts, potassium salts) and one or more detergents (*e.g.*, an ionic detergent, a non-ionic detergent). Suitable detergents for a wash buffer include, but are not limited to, sodium dodecyl sulfate (SDS), TRITON® X-100 detergent, TWEEN® 20 detergent, NP-40 detergent, or Igepal CA-630 detergent. Preferably, the wash buffer comprises one or more salts (*e.g.*, sodium citrate) having a total concentration of about 0.03M to about 0.09M and about 0.1% SDS. In a particular embodiment, the wash buffer comprises 1.864 mM NaOH and 2xSSC.

Exemplary wash conditions for room temperature in situ hybridization methods include, for example, an initial post-hybridization wash in 2x SSC for 5 min. at room temperature (e.g, about 21° C) followed by one or more additional washes in 0.03M to 0.09M monovalent salt (*e.g.*, SSC) and 0.1% SDS at room temperature for at least about 2 minutes per wash, preferably, in the range of about 2 minutes to about 5 minutes per wash.

In a particular embodiment, the step of removing unhybridized probes is carried out at room temperature. Exemplary conditions for removing unhybridized probes at room temperature are described, for example, in U.S. Patent Application Publication Nos. US 2013/0203055 A1 and US 2013/0149705 A1.

Detecting labels on chromosome-specific probes

The methods described herein further include the step of detecting labels on chromosome-specific probes that have hybridized to the prostate cells. Detection of the label on a chromosome-specific probe can be accomplished using an approach that is suitable for the particular label on the probe, and can be readily determined by those of ordinary skill in the art. For example, fluorophore labels can be detected by detecting the emission wavelength of the particular fluorophore used. Typical methods for detecting fluorescent signals include, *e.g.*, spectrofluorimetry, epifluorescence microscopy, confocal microscopy, and flow cytometry analysis. Fluorescent labels are generally preferred for detection of low levels of target because they provide a very strong signal with low background. Furthermore, fluorescent labels are optically detectable at high resolution and sensitivity through a quick scanning procedure, and different hybridization probes having fluorophores with different emission wavelengths (*e.g.*, fluorescein and rhodamine) can be used for a single sample to detect multiple target nucleic acids.

In the particular case of fluorescence *in situ* hybridization (FISH) procedures, which utilize fluorescent probes, a variety of different optical analyses can be utilized to detect hybridization complexes. Spectral detection methods are discussed, for example, in U.S. Patent No. 5,719,024; Schroeck et al. (Science 273:494-497, 1996); and Speicher et al. (Nature Genetics 12:368-375, 1996). Further guidance regarding general FISH procedures are discussed, for example, in Gall and Pardue (Methods in Enzymology 21:470-480, 1981); Henderson (International Review of Cytology 76:1-46, 1982); and Angerer et al. in Genetic Engineering: Principles and Methods (Setlow and Hollaender eds., Plenum Press, New York, 1985).

In one embodiment, the labels on the probes are indirect labels. Detection of indirect labels typically involves detection of a binding partner, or secondary agent. For example, indirect labels such as biotin and other haptens (*e.g.*, digoxigenin (DIG), DNP, or fluorescein) can be detected via an interaction with streptavidin (*i.e.*, in the case of biotin) or an antibody as the secondary agent. Following binding of the probe and target, the target-probe complex can be detected by using, *e.g.*, directly labeled streptavidin or antibody. Alternatively, unlabeled secondary agents can be used with a directly labeled "tertiary" agent that specifically binds to the secondary agent (*e.g.*, unlabeled anti-DIG antibody can be used, which can be detected with a labeled second antibody specific for the species and class of the primary antibody). The label for the secondary agent is typically a non-isotopic label, although radioisotopic labels can be used. Typical non-isotopic labels include, *e.g.*, enzymes and fluorophores, which can be conjugated to the secondary or tertiary agent. Enzymes commonly used in DNA diagnostics include, for example, horseradish peroxidase and alkaline phosphatase.

Detection of enzyme labels can be accomplished, for example, by detecting color or dye deposition (*e.g.*, p-nitrophenyl phosphate or 5-bromo-4-chloro-3-indolyl phosphate/nitroblue tetrazolium for alkaline phosphatase and 3,3'-diaminobenzidine-NiCl2 for horseradish peroxidase), fluorescence (*e.g.*, 4-methyl umbelliferyl phosphate for alkaline phosphatase) or chemiluminescence (*e.g.*, the alkaline phosphatase dioxetane substrates LumiPhos 530 from Lumigen Inc., Detroit Mich. or AMPPD and CSPD from Tropix, Inc.), depending on the type of enzymatic label employed. Chemiluminescent detection can be carried out with X-ray or Polaroid film or by using single photon counting luminometers (*e.g.*, for alkaline phosphatase labeled probes).

In certain embodiments, digital enhancement or integration is used to detect a signal from a label on a probe. For example, detection of the label can include the use of microscopic imaging using a CCD camera mounted onto the eyepiece tube of a microscope (*e.g.*, a binocular, monocular, or stereo microscope). In some embodiments, detection of the label is accomplished using image scanning microscopy. For example, recent advances in computerized image scanning microscopy have significantly increased the ability to detect rare cells using fluorescence microscopy, permitting detection of 1 positive cell in an environment of ~6x10$^5$ negative cells (see, *e.g.*, Mehes et al., Cytometry 42:357-362, 2000). Advanced image scanning software has been developed that can not only detect multiple colors but also fused or co-localized signals useful for, *e.g.*, detection of translocations on the DNA level (MetaSystems Group, Inc.) Scanning speed typically depends on the number of parameters utilized for reliable detection of single positive cells. Image scanning also allows for images of the cells scored positive to be manually examined for confirmation. Advanced image scanning software for automated, slide-based analysis has been developed that can not only detect multiple colors but also fused or co-localized signals useful for, *e.g.*, detection of translocations on the DNA level (MetaSystems Group, Inc.) Scanning speed typically depends on the number of parameters utilized for reliable detection of single positive cells. Automated slide-based scanning systems are particularly amenable to high throughput assays.

In one embodiment, scanning slide microscopy, *e.g.*, employing a MetaCyte Automated Bio-Imaging System (Meta System Group, Inc.), is used. This system consists of the following components: 1) Carl Zeiss Axioplan 2 MOT fluorescence microscope, 2) scanning 8-position stage, 3) PC Pentium III Processor, 4) Jai camera, 5) camera interface, 6) stage control, 7) trackball and mouse, and 8) printer. The focus analysis begins with a slide set-up loaded onto the microscope. The slide is scanned as the stage is moved and the image is captured. Following scanning of the entire slide, a gallery is created. Based on the criterion set up for positive or negative, the image analysis either results in a positive or negative signal. If negative, the slide is rescanned for rare event analyses. If positive, there is a filter change for the appropriate fluorescent signal and 5-7 planes are captured and analyzed. There is walk away/overnight operation for 8 slides (standard or 100 slides with optional tray changer). Adaptive detection algorithms and automatic exposure control function compensate for non-uniform staining conditions. Several markers can be detected simultaneously. The standard light source covers a wide spectrum from UV to IR. Scanning speed up to 1,000 cells per second can be used for rare cell detection if cellular fluorescent intensity allows detection in 1/1,000 sec. For strong signals, a lower magnification can be used to increase scanning speed.

Alternatively, detection of the probe can be performed in the absence of digital enhancement or integration.

Additional Method Steps

In some embodiments, the methods described herein can include one or more additional method steps that are generally employed in conventional *in situ* hybridization procedures (*e.g.*, sample pretreatment steps to make nucleic acids (*e.g.*, chromosomal DNA) in a sample more accessible to probes (*e.g.*, nucleic acid probes)). Such steps include, for example, fixing the prostate cells on a solid support, pretreating the prostate cells with at least one protease, denaturing the prostate cells, counterstaining the prostate cells.

In one embodiment, the sample (*e.g.*, prostate cells) can be fixed (*e.g.*, prior to the hybridization step). A variety of suitable fixatives are known in the art and include, for example, acid acetone solutions, various aldehyde solutions (*e.g.*, formaldehyde, paraformaldehyde, and glutaraldehyde) and acid alcohol solutions. Examples of specific fixatives for chromosomal preparations are discussed, for example, in Trask et al. (Science 230:1401-1402, 1985). The sample (*e.g.*, prostate cells) can be fixed in solution or on a solid support, such as, but not limited to, a microscope slide, a coverslip, a multiwell plate (*e.g.*, a microtitre plate), a fibrous matrix.

In another embodiment, the sample (*e.g.*, prostate cells) can be pretreated to make chromosomal DNA more accessible to probes. Such pretreatment can include, for example, treating the sample (*e.g.*, prostate cells) with one or more proteinases (*e.g.*, proteinase K, trypsin, pepsin, collagenase) and/or mild acids (*e.g.*, 0.02-0.2 N HCl, 25% to 75% acetic acid). A pretreatment with RNase can also be utilized to remove residual RNA from the biological sample. Other pre-treatment steps can include detergent permeabilization, heat denaturation, chemical denaturation and aging of the sample. In one embodiment, the sample can be denatured in a non-alkaline denaturation buffer (*e.g.*, 70% formamide) at an elevated temperature (*e.g.*, 72° C). In another embodiment, the sample can be denatured in a solution comprising at least one base (*e.g.*, NaOH) and at least one alcohol (*e.g.*, ethanol) at room temperature. Preferably, the base/alcohol solution contains about 0.07N base and about 70% ethanol.

In a further embodiment, chromosomal DNA in the sample (*e.g.*, prostate cells) can be counter-stained with a spectrally distinguishable DNA specific stain such as, for example, 4',6-diamidino-2-phenylindole (DAPI), propidium iodide (PI) or a Hoechst reagent/dye and mounted using an antifade reagent. The DNA stain can be added directly to the antifade reagent or can be incubated with the sample, drained and rinsed before the antifade reagent is added. Reagents and techniques for counterstaining and mounting samples are generally known in the art.

Other methods of diagnosing a prostate carcinoma

In a particular embodiment, the invention relates to a method of diagnosing whether a human subject has a prostate carcinoma as defined in the claims. Also disclosed herein is a method of diagnosing whether a human subject has a prostate carcinoma, comprising obtaining a sample comprising prostate cells from the subject; hybridizing a set of at least two chromosome-specific probes to the prostate cells, wherein each chromosome-specific probe has a detectable label and is specific for a different human chromosome selected from the group consisting of human chromosome 6, human chromosome 8, human chromosome 10 and human chromosome Y; removing unhybridized probes; detecting the labels on chromosome-specific probes that have hybridized to the prostate cells; and determining whether the prostate cells include prostate cells that are polysomic for at least one chromosome selected from the group consisting of chromosome 6, chromosome 8, chromosome 10 and chromosome Y, or a combination thereof, wherein the presence of prostate cells that are polysomic for at least one chromosome selected from the group consisting of chromosome 6, chromosome 8, chromosome 10 and chromosome Y, or a combination thereof, indicates that the subject has a prostate carcinoma.

Sets of chromosome-specific probes that are suitable for use in this method preferably include a chromosome-specific probe for human chromosome 6 and a chromosome-specific probe for human chromosome 10.

The set of chromosome-specific probes may include a chromosome-specific probe for human chromosome 6, a chromosome-specific probe for human chromosome 8, and a chromosome-specific probe for human chromosome 10.

The set of chromosome-specific probes may include a chromosome-specific probe for human chromosome 6, a chromosome-specific probe for human chromosome 8, a chromosome-specific probe for human chromosome 10, and a chromosome-specific probe for human chromosome Y.

The set of chromosome-specific probes may include a chromosome-specific probe for human chromosome 7, a chromosome-specific probe for human chromosome 16, a chromosome-specific probe for human chromosome 18, and a chromosome-specific probe for human chromosome 20. In the context of the present invention, the set of chromosome-specific probes are as defined in the claims.

Suitable samples for use in this method contain a sufficient number of prostate cells (*e.g.*, epithelial cells of prostatic origin), also referred to as prostatic cells, to allow for the detection of polysomic prostate cells. Examples of samples that contain prostate cells include, for example, ejaculate fluid, urine (*e.g.*, urine samples obtained following the application of pressure to a subject's prostate gland, as described herein above), and prostate tissue (*e.g.*, tissue from a biopsy obtained from a prostate gland). Preferably, the sample is ejaculate fluid.

Also disclosed herein is a method of diagnosing whether a human subject has a prostate carcinoma, comprising the steps of obtaining a sample containing prostate cells from the subject; hybridizing a combination of at least two, preferably three, more preferably four, chromosome-specific probes to the prostate cells, wherein each chromosome-specific probe has a detectable label and is specific for a different human chromosome (*e.g.*, human chromosome 1, 2, 3, 4, 5, 6, 7, 8, 9, 10, 11, 12, 13, 14, 15, 16, 17, 18, 19, 20, 21, 22, the human X chromosome or the human Y chromosome), and wherein the combination of probes results in an area under the curve (AUC) of at least 0.70 (*e.g.*, at least 0.80) for a receiver operating characteristic (ROC) curve that is produced when the combination is used to detect polysomic prostate cancer cells; removing unhybridized probes; detecting the labels on chromosome-specific probes that have hybridized to the prostate cells; and determining whether the prostate cells include prostate cells that are polysomic for human chromosomes that are recognized by one or more of the chromosome-specific probes in the combination. The presence of polysomic prostate cells that are recognized by one or more of the chromosome-specific probes in the combination indicates that the subject has a prostate carcinoma.

Preferably, the combination includes three chromosome-specific probes. In another embodiment, the combination includes four chromosome-specific probes. In other embodiments, the combination can include more than four (*e.g.*, five, six, seven, eight, nine, ten, eleven, twelve) chromosome-specific probes.

The combination of probes can result in an area under the curve (AUC), calculated according to standard statistical methods known in the art, of at least 0.70 (*e.g.*, at least 0.75, at least 0.80) for a receiver operating characteristic (ROC) curve, also calculated according to standard statistical methods known in the art, that is produced when the combination is used to detect polysomic prostate cancer cells. Preferably, the combination of probes result in an area under the curve (AUC), calculated according to standard statistical methods, of at least 0.85, more preferably at least 0.88, even more preferably at least 0.90.

Suitable samples for use in this method contain a sufficient number of prostate cells (*e.g.*, epithelial cells of prostatic origin) to allow for the detection of polysomic prostate cells and include, for example, the exemplary sample types described herein.

Method of differentiating a high grade prostate cancer from a low grade prostate cancer

In another embodiment, the disclosure relates to a method of differentiating a high grade prostate cancer from a low grade prostate cancer in a subject having a prostate carcinoma as defined in the claims. Also disclosed herein is a method of differentiating a high grade prostate cancer from a low grade prostate cancer in a human subject having a prostate carcinoma. In a disclosure the method of differentiating a high grade prostate cancer from a low grade prostate cancer comprises the steps of obtaining a sample containing prostate cells from the subject; hybridizing a set of at least two chromosome-specific probes to the prostate cells, wherein at least one chromosome-specific probe has a detectable label and is specific for a human chromosome selected from the group consisting of human chromosome 7 and human chromosome Y; removing unhybridized probes; detecting the labels on chromosome-specific probes that have hybridized to the prostate cells; and determining whether the prostate cells include prostate cells that are polysomic for at least one chromosome selected from the group consisting of chromosome 7 and chromosome Y, or a combination thereof. The presence of prostate cells that are polysomic for at least one chromosome selected from the group consisting of chromosome 7 and chromosome Y, or a combination thereof, indicates that the subject has a high grade prostate cancer.

As used herein, a "high grade prostate cancer" refers to a prostate cancer that, if observed pathologically (*e.g.*, under a microscope), would be assigned a Gleason score of greater than 6 by a person of skill in the art.

In contrast, a "low grade prostate cancer" refers to a prostate cancer that, if observed pathologically, would be assigned a Gleason score of 6 or less than 6 by a person of skill in the art.

Sets of chromosome-specific probes that are suitable for the method of differentiating a high grade prostate cancer from a low grade prostate cancer preferably include a chromosome-specific probe for human chromosome 7 and a chromosome-specific probe for human chromosome 20. In the context of the present invention, the set of chromosome-specific probes are as defined in the claims.

The set of chromosome-specific probes may include a chromosome-specific probe for human chromosome 6, a chromosome-specific probe for human chromosome 8, a chromosome-specific probe for human chromosome 10, and a chromosome-specific probe for human chromosome Y.

The set of chromosome-specific probes may include a chromosome-specific probe for human chromosome 7, a chromosome-specific probe for human chromosome 16, a chromosome-specific probe for human chromosome 18, and a chromosome-specific probe for human chromosome 20. In the context of the present invention, the set of chromosome-specific probes are as defined in the claims.

Samples that are suitable for the method of differentiating a high grade prostate cancer from a low grade prostate cancer include, for example, ejaculate fluid, urine (*e.g.*, voided urine obtained following the application of pressure to a subject's prostate gland, as described herein above), and prostate tissue (*e.g.*, tissue from a biopsy obtained from a prostate gland). Preferably, the sample is ejaculate fluid.

Kits for Detecting Prostate Cancer Cells

The present disclosure also provides kits for detecting prostate cancer cells in a sample from a human subject as defined in the claims. Also disclosed herein are kits for detecting prostate cancer cells (*e.g.*, polysomic prostate cancer cells) in a sample from a human subject. In some embodiments, the kits comprise at least two chromosome-specific probes that have a detectable label and are specific for a different human chromosome selected from the group consisting of human chromosome Y, human chromosome 6, human chromosome 7, human chromosome 8, human chromosome 10, human chromosome 13, human chromosome 16, human chromosome 18 and human chromosome 20.

The kits may include any combination, or set, of at least two chromosome-specific probes that are specific for different human chromosomes selected from the group consisting of human chromosome Y, human chromosome 6, human chromosome 7, human chromosome 8, human chromosome 10, human chromosome 13, human chromosome 16, human chromosome 18 and human chromosome 20. Exemplary combinations/sets of chromosome-specific probes include, for example, a set of probes containing a chromosome-specific probe for human chromosome 6 and a chromosome-specific probe for human chromosome 10; and a set of probes containing a chromosome-specific probe for human chromosome 7 and a chromosome-specific probe for human chromosome 20.

The kit may contain at least two (*e.g.*, two, three, four, five, six, seven or eight) chromosome-specific probes, wherein each probe is specific for a different human chromosome. Preferably, the human chromosome is selected from the group consisting of human chromosome Y, human chromosome 6, human chromosome 7, human chromosome 8, human chromosome 10, human chromosome 13, human chromosome 16, human chromosome 18 and human chromosome 20.

In one disclosure, a kit comprises a probe set consisting of two chromosome-specific probes, or, a kit comprises a probe set consisting of three chromosome-specific probes, or, a kit comprises a probe set consisting of four chromosome-specific probes.

The kits described herein may contain a chromosome-specific probe for human chromosome 6 and a chromosome-specific probe for human chromosome 10. In the context of the present invention, the set of chromosome-specific probes are as defined in the claims.

A kit may include a chromosome-specific probe for human chromosome 6, a chromosome-specific probe for human chromosome 8, and a chromosome-specific probe for human chromosome 10.

A kit may include a chromosome-specific probe for human chromosome 6, a chromosome-specific probe for human chromosome 8, a chromosome-specific probe for human chromosome 10, and a chromosome-specific probe for human chromosome Y.

A kit may include a chromosome-specific probe for human chromosome 6, a chromosome-specific probe for human chromosome 8, a chromosome-specific probe for human chromosome 10, and a chromosome-specific probe for human chromosome 18.

A kit may include a chromosome-specific probe for human chromosome 7 and a chromosome-specific probe for human chromosome 20.

A kit may include a chromosome-specific probe for human chromosome 7, a chromosome-specific probe for human chromosome 16, a chromosome-specific probe for human chromosome 18, and a chromosome-specific probe for human chromosome 20.

A kit may include a chromosome-specific probe for human chromosome Y, a chromosome-specific probe for human chromosome 7, a chromosome-specific probe for human chromosome 10, and a chromosome-specific probe for human chromosome 20.

In a disclosure, the invention relates to a kit for detecting prostate cancer cells in a sample from a human subject, comprising a combination of at least two, preferably three, more preferably four, chromosome-specific probes, wherein each chromosome-specific probe has a detectable label and is specific for a different human chromosome (*e.g.*, human chromosome 1, 2, 3, 4, 5, 6, 7, 8, 9, 10, 11, 12, 13, 14, 15, 16, 17, 18, 19, 20, 21, 22, the human X chromosome or the human Y chromosome), and wherein the combination of probes results in an area under the curve (AUC) of at least 0.70 (*e.g.*, at least 0.80) for a receiver operating characteristic (ROC) curve that is produced when the combination is used to detect polysomic prostate cancer cells.

Preferably, the combination includes three chromosome-specific probes. In another embodiment, the combination includes four chromosome-specific probes. In other embodiments, the combination can include more than four (*e.g.*, five, six, seven, eight, nine, ten, eleven, twelve) chromosome-specific probes.

The combination of probes in the kit can result in an area under the curve (AUC), calculated according to standard statistical methods known in the art, of at least 0.70 (*e.g.*, at least 0.75, at least 0.80) for a receiver operating characteristic (ROC) curve, also calculated according to standard statistical methods known in the art, that is produced when the combination is used to detect polysomic prostate cancer cells. Preferably, the combination of probes result in an area under the curve (AUC), calculated according to standard statistical methods, of at least 0.85, more preferably at least 0.88, even more preferably at least 0.90.

Suitable probes for inclusion in the kits of the disclosure include any probe described herein as being suitable for use in the methods of diagnosing whether a human subject has a prostate carcinoma. In one embodiment, the probes in a kit are nucleic acid probes (*e.g.*, DNA probes). In a further embodiment, the nucleic acid probes a single-stranded DNA probes. Preferably, the probes are oligonucleotide probes (*e.g.*, single-stranded DNA oligonucleotide probes).

In one embodiment, each probe in a kit has a detectable label that differs from the detectable labels on the other probes in the kit. In another embodiment, each probe in the kit has the same detectable label. Preferably, the probes in the kit have a direct label. In a particular embodiment, the direct label is a fluorescent label (*e.g.*, a fluor).

In some embodiments, the probes in the kit can be attached to, affixed to, deposited on, embedded in, or sorbed to a matrix. Preferably, the matrix is a fibrous matrix. The fibrous matrix can be composed of a naturally-occurring fiber or a synthetic fiber. The fiber can be a woven fiber or a nonwoven fiber. Exemplary fibers include, but are not limited to, glass fibers, wool fibers, and plant fibers. In a preferred embodiment, the fiber is a glass fiber. In another embodiment, the fibrous matrix is made from a cellulose-based material (*e.g.*, a cellulose fiber). Suitable cellulose-based materials include, but are not limited to, cellulose, nitrocellulose, carboxymethyl-cellulose, rayon, and viscose. In a particular embodiment, the dry fibrous matrix is a filter paper (*e.g.*, a cellulose-based filter paper, a glass fiber filter paper). Suitable filter papers are available commercially, including, for example, Whatman™ cellulose and glass microfiber filter papers (GE Healthcare). Methods of preparing fibrous matrices containing nucleic acids are known in the art.

The kits described herein can include, in some embodiments, one or more additional components as defined in the claims. Such additional components may include, for example, reagents for performing *in situ* hybridization (*e.g.*, a protease, a fixative, a denaturation buffer, a hybridization buffer, a wash buffer, a secondary detection reagent, a stain for chromosomal DNA (*e.g.*, DAPI, Hoechst reagent), an antifade reagent, instructions, protocols, or a combination thereof).

The kits described herein can further include a denaturation buffer. In one embodiment, the denaturation buffer can include formamide (*e.g.*, 70% formamide). In an alternative embodiment, the denaturation buffer can include a base (*e.g.*, NaOH) and an alcohol (*e.g.*, ethanol). Exemplary bases for use in the denaturation buffer include, for example, potassium hydroxide, barium hydroxide, caesium hydroxide, sodium hydroxide, strontium hydroxide, calcium hydroxide, lithium hydroxide, rubidium hydroxide, magnesium hydroxide, butyl lithium, lithium diisopropylamide, lithium diethylamide, sodium amide, sodium hydride, lithium bis(trimethylsilyl)amide, sodium carbonate and ammonia, or a combination thereof. Preferably, the base is an alkali base. More preferably, the base is sodium hydroxide. Exemplary alcohols for use in the denaturation buffer include, for example, ethanol, methanol, propanol, butanol, pentanol and isoamyl alcohol, among others, or mixtures thereof.

Preferably, denaturation buffers containing a base and an alcohol include about 0.03N to about 0.17N base, for example, about 0.05N, about 0.06N, about 0.07N, about 0.08N, about 0.09N or about 0.1N base. Preferably, the denaturation buffer comprises about 0.07N NaOH (*i.e.*, 0.07M NaOH). The denaturation buffer further includes at least one alcohol at a concentration of about 50% to about 90% by volume, for example about 60%, about 70% or about 80% by volume. Preferably, the alcohol is present at a concentration of about 70% by volume. In a particular embodiment, the denaturation buffer comprises about 70% ethanol. In yet another embodiment, the denaturation buffer comprises formamide (*e.g.*, 70% formamide) instead of alcohol or base. Exemplary alkaline denaturation buffers containing alcohol are described, for example, in U.S. Patent Application Publication No.: US 2013/0149705, the contents of which are incorporated herein by reference.

The kits described herein can also include a hybridization buffer. In one embodiment, the hybridization buffer comprises formamide and one or more salts (*e.g.*, sodium salts) at a final concentration of about 0.03M to about 0.09M instead of a base. Preferably, the one or more salts include sodium citrate. Other suitable salts for use in the hybridization buffer include sodium chloride. In another embodiment, the hybridization buffer has a pH of in the range of about 10 and 13, preferably between about 11 and 12, and includes one or more bases (*e.g.*, NaOH). Any of the bases described herein as being suitable for use in the denaturation solution can also be used in the hybridization buffer. In addition, the hybridization buffer containing a base can further include formamide (*e.g.*, about 20-60% formamide) and dextran sulfate (*e.g.*, about 5-40% dextran sulfate). In one embodiment, the hybridization buffer contains about 30-50% formamide, about 10% dextran sulfate and about 1-3 mM NaOH, and has a pH in the range of about 11 to about 12. In another embodiment, the hybridization buffer contains about 4.2 mM NaOH, about 42% formamide and about 28% dextran sulfate.

The kits of the invention can further include one or more wash buffers. Suitable wash buffers typically contain one or more salts (*e.g.*, sodium salts, lithium salts or potassium salts) at a final concentration of about 0.03M to about 0.09M. In a particular embodiment, the wash buffer includes sodium citrate and sodium chloride. The wash buffers can further comprise a detergent including, but not limited to, sodium dodecyl sulfate (SDS). Suitable concentrations of SDS in the wash buffers are typically in the range of about 0.01% to about 1.0% SDS, preferably about 0.1% SDS. In addition, wash buffers can optionally include formamide. In one embodiment the kit includes a wash buffer comprising 1.864 mM NaOH and 2xSSC.

Alternatively, the wash buffer can contain one or more bases (*e.g.*, NaOH) and have a pH of between about 10 and 13, preferably between about 11 and 12. Any of the bases described herein as being suitable for use in the denaturation and hybridization buffers can also be used in the wash buffer. Preferably, the one or more wash buffers include about 1×-5×SSC and about 1-10 mM base. In a particular embodiment, the wash buffer contains about 2×SSC and about 1.75 mM NaOH, and has a pH of about 11. In another embodiment, the wash buffer contains about 2×SSC and about 3 mM NaOH.

The kit can also include one or more reagent(s) for detecting labeled probes. In one embodiment, the kit includes a secondary agent (*e.g.*, streptavidin labeled with a fluorophore) for detecting an indirect label (*e.g.*, biotin) on a probe.

Typically, the kits are compartmentalized for ease of use and can include one or more containers with reagents. In one embodiment, all of the kit components are packaged together. Alternatively, one or more individual components of the kit can be provided in a separate package from the other kits components.

A description of example embodiments of the invention follows.

EXAMPLE 1: DETECTION OF POLYSOMIC PROSTATE CANCER CELLS IN URINE SAMPLES USING OLIGOFISH-BASED METHODS

MATERIALS AND METHODS

Informed Consent: The study protocol was approved by the Western Institutional Review Board (WIRB). All subjects were informed of the procedures and risks of the study and provided their informed consent before receiving the digital rectal examination (DRE) with pressure.

Obtaining the urine sample: On the day of biopsy, the urologist performed a DRE with pressure on the subject. Prostate lobes were stroked with the physician's finger through the rectum 3 times, with approximately 1 cm depression. The lobes were stroked from base to apex and from the lateral to the median line. Voided urine was collected and immediately preserved in PreservCyte at 2:1 ratio and kept at +4°C until processed. A total of 49 patient urine samples were used in this study.

Urine Processing and preparing the slides: Urine was centrifuged in order to pellet the cells. The cells were rinsed with CYTOLYT® solution and centrifuged again. The cells were re-suspended in 20 mL PRESERVCYT® solution in order to prepare the slides with the THINPREP® machine. Slides were fixed in methanol:acetic and aged 15 minutes at 75 °C before storing them in hermetic boxes kept at -20 °C with silica gel.

OligoFISH procedure: The slides were pretreated with protease at room temperature for 5 minutes followed by a 5-minute detergent treatment. The slides were then slightly fixed in 1% formalin. After rinsing in 1xPBS cellular DNA was denatured at room temperature for 5 minutes in a denaturing solution. The slides were then hybridized for 10 minutes with chromosome-sepcific OLIGOFISH® probes (Cellay, Inc., Cambridge, MA) to detect chromosomes Y, 6, 8 and 10. The coverslips were floated in 2xSSC under agitation for 5 minutes. Then the slides were washed at room temperature for 5 minutes with a wash buffer. Finally the slides were rinsed in 2xSSC before being mounted with Antifade and 4',6-diamidino-2-phenylindole (DAPI) before being examined under the fluorescence microscope.

Scoring of the slides: 500 cells were scored for each specimen. The only cells excluded from the analysis were polymorph nucleated white blood cells that are easily recognizable. Once the slides were scored they were washed to remove coverslips and Antifade and denatured again 5 minutes to strip the probes. Then they were hybridized with the second probe panel for the detection of chromosomes 7, 16, 18 and 20 and 500 cells were also scored.

Determining the presence of prostatic (e.g., polysomic) cells in the urine samples: Slides from 10 subjects were processed without the pretreatments in order to keep the cytoplasm intact. Prostatic cells were identified by morphology since they have a high nuclear cytoplasmic ratio with an eccentric nucleus while the urothelial cells have a very low nuclear cytoplasmic ratio and are much bigger cells sometimes bi-nucleated. White blood cells were not scored since they can be recognized by the polylobulated nucleus.

Statistical analysis

Calculating the results for each sample: For all cases scored, the percentage of cells with chromosomal loses, chromosomal gains and aneuploidy were calculated with the margin of error at 95 % confidence using the following formula:

$$margin\ of\ error = z\sqrt{\frac{p(1-p)}{n}}$$

Where z is equal to 1.645 at 95 % confidence, p is the frequency and n the number of cells scored.

Analytical performance of the probes: Analytical sensitivity and specificity were calculated as recommended by the American College of Genetic Medicine as follows:

Analytical sensitivity for each chromosomal probe was calculated as the percentage of cells with the correct number of signals when scoring 200 cells from 5 chromosomally normal males.

Analytical specificity for each chromosomal probe was calculated as the percentage of metaphases showing the signals in the correct locus when scoring 200 metaphases from 5 chromosomally normal males.

Clinical performance of the assay: ROC curves were calculated by varying the cutoff value from having clinical specificity equal to 1 (100 %) and sensitivity equal to 0 to specificity equal to 0 and sensitivity equal to 1. The values of true positive rates or clinical sensitivity were plotted against false positive rates or 1-Specifity and the area under the curve was calculated using GraphPad Prism 6 software (GraphPad Software, San Diego, CA).

Cutoff was determined using the same values as in the ROC curves but plotting sensitivity against specificity. The best cut off value was determined as the cutoff where sensitivity and specificity intersect.

Clinical sensitivity was calculated as the percentage of patients that showed cancer by histology that had a positive test result:

$$Clinical\ Sensitivity = 100 \times \frac{True\ positives}{True\ positives + False\ negatives}$$

Clinical specificity was calculated as the percentage of patients not showing cancer by cystoscopy and/or histology that had a negative test result.

$$Clinical\ Specificity = 100 \times \frac{True\ negatives}{True\ negatives + False\ positives}$$

Positive prediction value (PPV) was calculated as the probability of having cancer if the subject has a positive test result.

$$PPV = 100 \times \frac{True\ positives}{True\ positives + False\ positives}$$

Negative prediction values (NPV) was calculated as the probability of having cancer if the subject has a positive test result.

$$NPV = 100 \times \frac{True\ negatives}{True\ negatives + False\ negatives}$$

Accuracy was calculated as the percentage of concordance between the biopsy result in cystoscopy/pathology and the test results.

$$Accuracy = 100 \times \frac{True\ positives + True\ negatives}{Total\ number\ of\ cases}$$

RESULTS

Analytical performance of the test

Analytical specificity

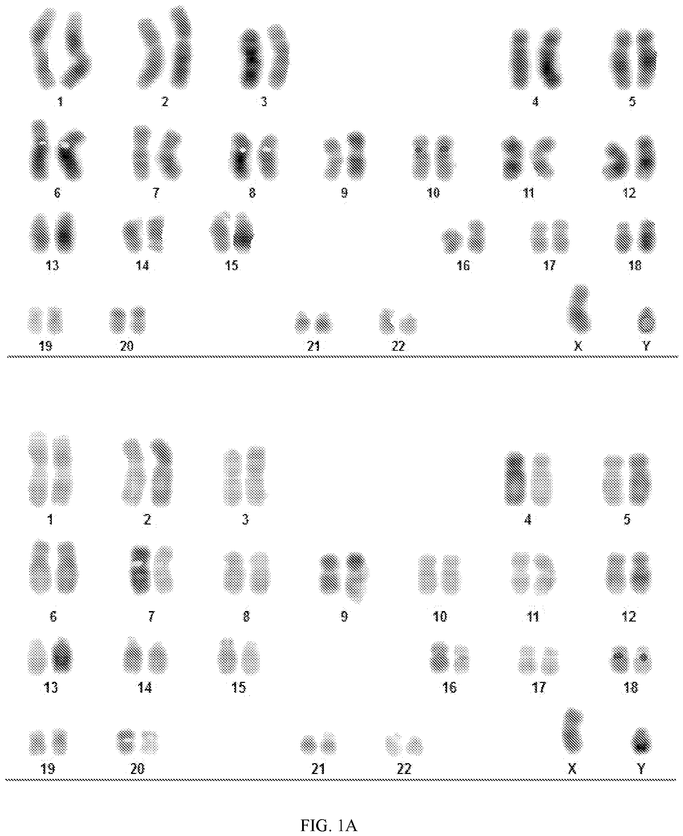

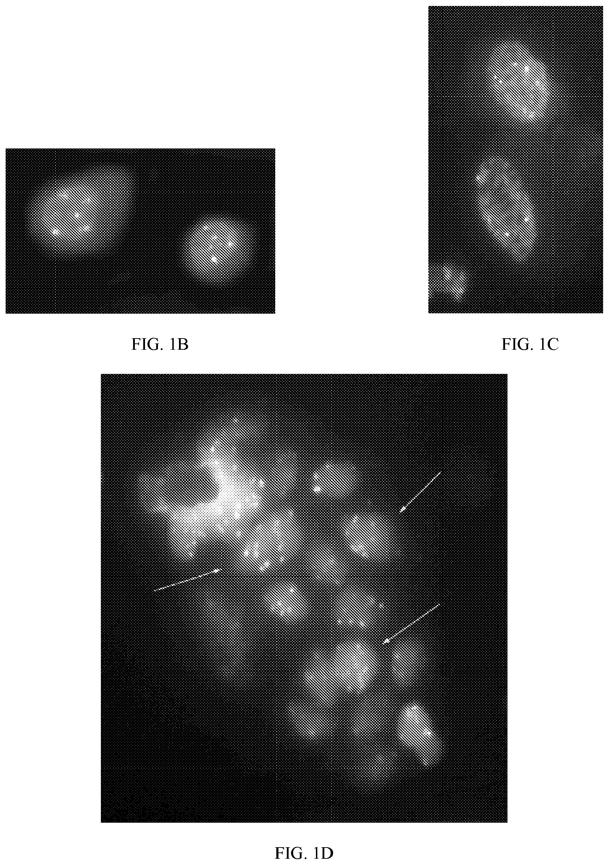

All eight probes used in the study hybridized to the correct locus in all the metaphases analyzed showing 100 % analytical specificity. Examples of karyotyped metaphases for certain chromosome-specific probes are shown in FIG. 1.

Analytical Sensitivity

All the probes showed >98 % analytical sensitivity as recommended by the American College of Genetic Medicine (Table 1).

Table 1. Analytical sensitivity of the OLIGOFISH® probes

| Chromosome probe | Fluor | Analytical Sensitivity (%) | Margin of error 95% % |
|---|---|---|---|
| Yq12 | Green | 99.5 | 0.95 |
| 6 | Aqua | 99.5 | 0.95 |
| 8 | Gold | 99.5 | 0.95 |
| 10 | Red | 99.1 | 1.30 |
| | | | |
| 7 | Aqua | 99.0 | 1.38 |
| 16 | Green | 99.0 | 1.38 |
| 18 | Red | 98.5 | 1.7 |
| 20 | Gold | 98.0 | 1.9 |

Presence of prostate cells in urine samples

The first 10 patient samples were hybridized without the pretreatments in order to preserve the cytoplasm. Prostate cells were identified by their distinct morphology from the urothelial cells. The percentage of prostatic cells in each sample is shown in Table 2.

Table 2. Percentage of prostate cells in urine after DRE with pressure.

| Subject | Cohort | prostate cells | total cells | % | MoE 95% |
|---|---|---|---|---|---|
| 01-001 | HG | 178 | 266 | 66.9 | 5.7 |
| 01-002 | Benign | 356 | 500 | 71.2 | 4.0 |
| 01-004 | Benign | 413 | 500 | 82.6 | 3.3 |
| 01-005 | Benign | 181 | 451 | 40.1 | 4.5 |
| 01-006 | Benign | 364 | 500 | 72.8 | 3.9 |
| 01-007 | Benign | 254 | 500 | 50.8 | 4.4 |
| 01-009 | LG | 161 | 228 | 70.6 | 5.9 |
| 01-011 | LG | 126 | 287 | 43.9 | 5.7 |
| 01-013 | LG | 390 | 500 | 78.0 | 3.6 |
| 01-014 | HG | 368 | 500 | 73.6 | 3.9 |

(continued)

| Subject | Cohort | prostate cells | total cells | % | MoE 95% |
|---------|--------|----------------|-------------|------|---------|
|  |  |  | Average | 65.1 |  |
|  |  |  | SD | 14.7 |  |
|  |  |  | SeM 95% | 9.1 |  |
|  |  |  | Min | 40.1 |  |
|  |  |  | Max | 82.6 |  |

Clinical performance of the assay

Chromosomal loses and chromosomal gains

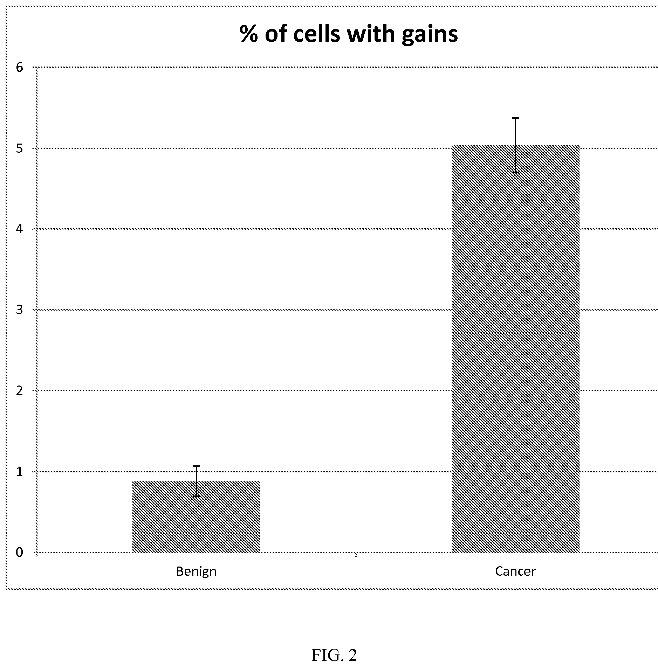

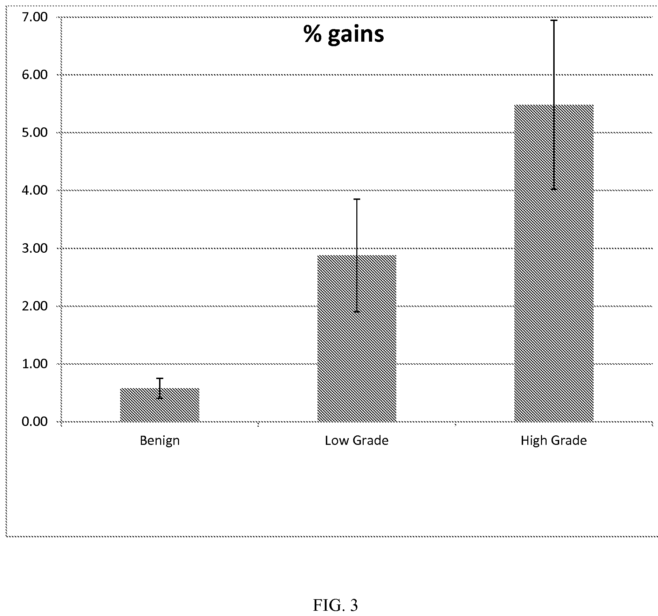

Chromosomal losses, especially loss of chromosome Y, were observed in all cohorts including benign lesions and, therefore, were not informative for diagnostic purposes. However, there were clear differences in the percentages of cells with chromosome gains between the benign, low grade cancer and high grade cancer samples (FIGS. 2 and 3).

After scoring 500 cells in each sample for both probe sets after each hybridization when possible, the results for each subject were pulled together in order to analyze the clinical utility of the 8 chromosomes together. Clinical Sensitivity and Specificity were calculated at every cut off for them to vary from 0 % to 100% or from 100% to 0%. Sensitivity was calculated, the ROC curves were plotted and the area under the curve (AUC) was calculated according to standards methods. Standard methods of computing the AUC for ROC curves are well known and are described, for example, in EP24-A2, Assessment of the Diagnostic Accuracy of Laboratory Tests Using the Receiver Operating Characteristic Curves; Approved Guideline - Second Edition, November 2011 of the Clinical and Laboratory Standards Institute.

Benign vs. Cancer

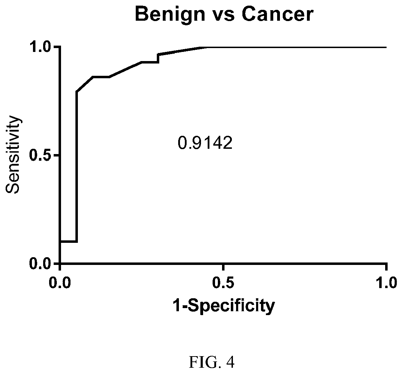

After measuring the area under the curve for the benign vs. cancer samples for all 8 chromosomes, a calculation of 0.93 was obtained, which corresponds to an excellent test in differentiating Benign vs. Cancer (FIG. 4).

ROC curves for the individual chromosomes

In order to identify the chromosomes that are the most informative, clinical sensitivity and specificity were calculated by looking at the results of only one chromosomal probe. ROC curves were then plotted and area under the curve was used to determine which individual chromosomes and combinations of chromosomes were most informative (Table 3).

Table 3. Areas under the curve for 8 individual chromosomes and specific combinations of 2, 3, or 4 chromosomes for differentiating between benign and cancerous samples.

| chromosome | AUC | best cutoff | specificity | sensitivity |
|------------|--------|-------------|-------------|-------------|
| 6 | 0.8644 | 0.75 | 0.86 | 0.79 |
| 10 | 0.8617 | 0.50 | 0.76 | 0.90 |
| 8 | 0.8601 | 0.50 | 0.76 | 0.90 |
| 18 | 0.8569 | 0.50 | 0.67 | 0.96 |
| 7 | 0.8408 | 1.00 | 0.83 | 0.77 |
| 16 | 0.8360 | 0.75 | 0.83 | 0.80 |
| Y | 0.8226 | 1.25 | 0.81 | 0.83 |
| 20 | 0.8077 | 1.50 | 0.94 | 0.73 |
| 6, 10 | 0.8688 | 0.75 | 0.75 | 0.83 |
| 6, 10, 8 | 0.8562 | 0.75 | 0.70 | 0.83 |

(continued)

| chromosome | AUC | best cutoff | specificity | sensitivity |
|---|---|---|---|---|
| 6, 10, 8, Y | 0.8841 | 1.25 | 0.75 | 0.96 |
| 7, 16, 18, 20 | 0.8898 | 1.50 | 0.88 | 0.96 |

The chromosome with the highest AUC was chromosome 6 followed by chromosome 10, 8 and 18, respectively.

ROC curves for specific combinations of chromosomes

After taking into account the results obtained with the 8 individual chromosomes, ROC curves were plotted and the areas under the curve were determined for the following combinations: chromosomes 6 and 10; chromosomes 6, 10 and 8; chromosomes 6, 10, 8 and Y; and chromosomes 7, 16, 18 and 20. Three out of four combinations tested resulted in ROC curves that were higher than any of the individual chromosomes tested, with the two 4-probe combinations (chromosomes 6, 10, 8 and Y: chromosomes 7, 16, 18 and 20) yielding the highest AUC values.

Clinical performance of the test

Cut off determination

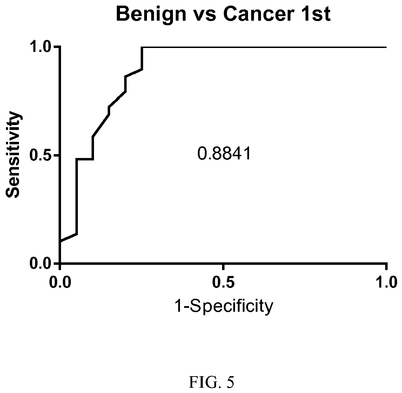

Taking into account the combination of chromosomes 6, 10, 8 and Y, Sensitivity against Specificity was plotted after varying the cutoff as for the ROC curve calculation. The best cutoff value should appear at the intersection of both (FIG. 5).

The cutoff at the crossing of Specificity and Sensitivity was 1.75 %. Using this cut off the clinical performance of the test in differentiating benign from cancer was calculated with the following results.

Sensitivity = 90 %
Specificity = 90 %
PPV = 95 %
NPV = 82 %
Accuracy = 90 %

Distinguishing Low Grade from High Grade Prostate Carcinomas

To determine whether the diagnostic test would also predict the Gleason score of a prostate tumor, the results of the test were compared to the pathology findings. ROC curves were calculated as described herein.

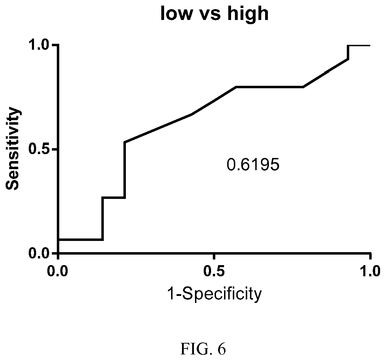

As shown in FIG. 6, the area under the curve using all 8 chromosomes tested was only 0.66, which corresponds to a poor test. However, since each chromosome could be participating differently, each chromosome was analyzed to determine which individual chromosomes were most informative.

Table 4. Areas under the curve for 8 individual chromosomes and specific combinations of 2 or 4 chromosomes for distinguishing high from low grade tumors.

| chromosome | AUC | best cutoff | specificity | sensitivity |
|---|---|---|---|---|
| Y | 0.6103 | 2.75 | 0.80 | 0.57 |
| 7 | 0.6052 | 2.75 | 0.69 | 0.61 |
| 20 | 0.6012 | 2.50 | 0.85 | 0.54 |
| 10 | 0.5761 | 2.75 | 0.80 | 0.57 |
| 6 | 0.5742 | 2.00 | 0.60 | 0.57 |
| 18 | 0.5466 | 3.00 | 0.61 | 0.54 |
| 8 | 0.5387 | 2.50 | 0.73 | 0.57 |
| 16 | 0.4512 | 2.50 | 0.77 | 0.36 |
| 7,20 | 0.5575 | 3.00 | 0.60 | 0.69 |

(continued)

| chromosome | AUC | best cutoff | specificity | sensitivity |
|---|---|---|---|---|
| Y, 6, 10, 8 | 0.6065 | 5.00 | 0.79 | 0.60 |
| 7, 16, 18, 20 | 0.5919 | 4.25 | 0.61 | 0.71 |

Clinical performance

Cut-off determination

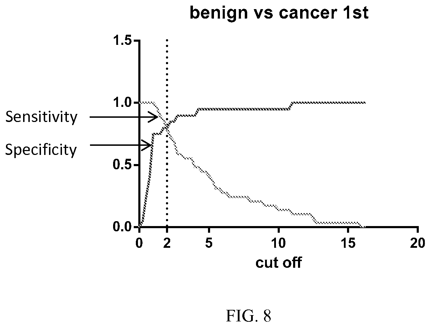

In order to calculate the best cut off value, Sensitivity vs. Specificity was plotted for the combination of chromosomes 6, 10, 8 and Y while changing the cut off value to find where the two values cross (FIG. 8). As seen in FIG. 8 the two lines crossed each other in between two cut off values, and all clinical performance values were calculated at these two values (Table 5).

Table 5. Clinical performance of the test in differentiating low grade from high grade at the two best cut off values.

| Cut off value | 3.75 % | 4% |
|---|---|---|
| Clinical Specificity | 67% | 78 % |
| Clinical Sensitivity | 73 % | 64 % |
| PPV | 73 % | 78 % |
| NPV | 67 % | 64 % |
| Accuracy | 70 % | 70 % |

EXAMPLE 2: EXPANDED STUDY - DETECTION OF POLYSOMIC PROSTATE CANCER CELLS IN URINE SAMPLES USING OLIGOFISH-BASED METHODS

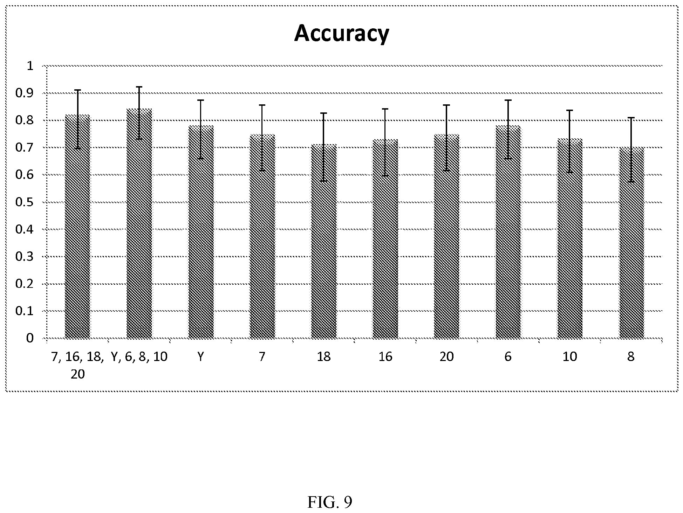

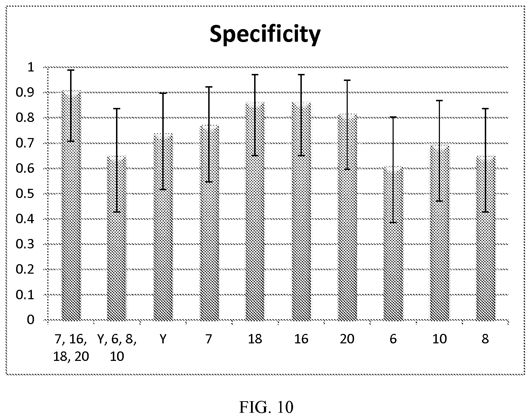

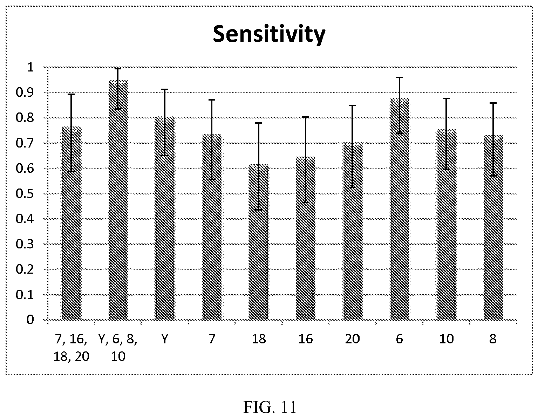

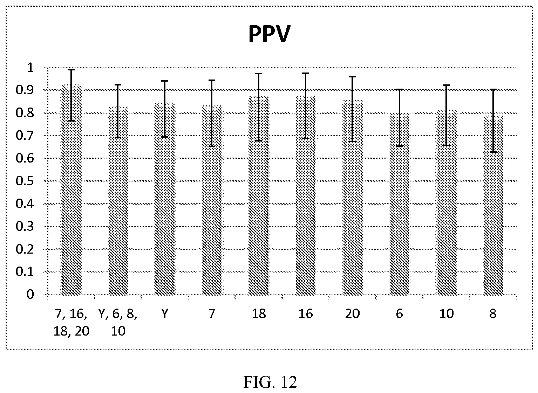

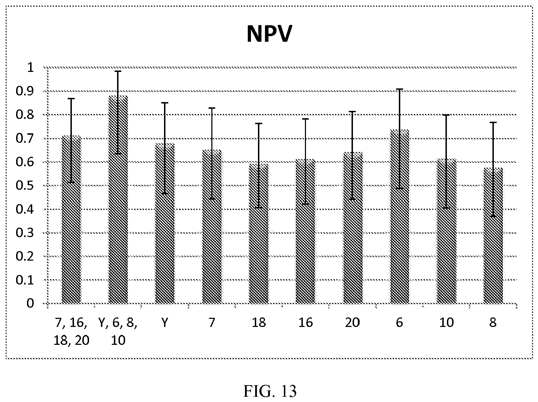

The study described in Example 1 was expanded to include 64 patients. The results are presented in Tables 6 and 7, and FIGS. 9-13. Although statistical differences were not observed, the panel of probes specific for chromosomes 7, 16, 18 and 20 performed better than the other panel and the various individual chromosomes tested (Table 6). Based on AUC values for the individual chromosomes alone, a panel of probes specific for chromosomes 7, 16, 18 and Y is predicted to perform best (Table 6).

Table 6. Areas under the curve (AUC) for 8 individual chromosomes and two specific combinations of 4 chromosomes for differentiating between benign and cancerous samples.

| Probes | AUC |
|---|---|
| 7, 16, 18, 20 | 0.8408 |
| Y, 6, 8, 10 | 0.8201 |
| Y | 0.8099 |
| 7 | 0.7872 |
| 18 | 0.7789 |
| 16 | 0.7718 |
| 20 | 0.7711 |
| 6 | 0.7642 |
| 10 | 0.7535 |
| 8 | 0.7277 |

Table 7. Clinical performance of 8 individual chromosomes and two specific combinations of 4 chromosomes in differentiating low grade from high grade tumors. (PPV: positive prediction value; NPV: negative prediction value; AUC: area under the curve).

| Probe | Specificity | Sensitivity | PPV | NPV | Accuracy | AUC |
|---|---|---|---|---|---|---|
| 7 | 0.80 | 0.58 | 0.79 | 0.60 | 0.68 | 0.64 |
| 6 | 1.00 | 0.35 | 1.00 | 0.55 | 0.63 | 0.61 |
| 10 | 0.89 | 0.43 | 0.83 | 0.55 | 0.63 | 0.61 |
| 18 | 0.73 | 0.47 | 0.69 | 0.52 | 0.59 | 0.60 |
| 20 | 0.87 | 0.42 | 0.80 | 0.54 | 0.62 | 0.60 |
| 8 | 0.83 | 0.43 | 0.77 | 0.54 | 0.61 | 0.60 |
| Y, 6, 8, 10 | 0.94 | 0.30 | 0.88 | 0.52 | 0.59 | 0.58 |
| 7, 16, 18, 20 | 0.80 | 0.47 | 0.75 | 0.55 | 0.62 | 0.56 |
| Y | 0.72 | 0.52 | 0.71 | 0.54 | 0.61 | 0.56 |
| 16 | 0.80 | 0.47 | 0.75 | 0.55 | 0.62 | 0.52 |

## Claims

1. A method of diagnosing whether a human subject has a prostate carcinoma, comprising:

   a) providing a sample containing prostate cells from the subject;
   b) hybridizing a set of at least four chromosome-specific probes to the prostate cells, wherein each chromosome-specific probe has a detectable label and a nucleotide sequence that is complementary to a repetitive genomic sequence of a specific human chromosome, and is specific for a different human chromosome; wherein the set of chromosome-specific probes includes

   i. a chromosome-specific probe for human chromosome 6, a chromosome-specific probe for human chromosome 8, a chromosome-specific probe for human chromosome 10, and a chromosome-specific probe for human chromosome Y; or,
   ii. a chromosome-specific probe for human chromosome 7, a chromosome-specific probe for human chromosome 16, a chromosome-specific probe for human chromosome 18, and a chromosome-specific probe for human chromosome 20;

   c) removing unhybridized probes;
   d) detecting the labels on chromosome-specific probes that have hybridized to the prostate cells; and
   e) determining whether the prostate cells include prostate cells that are polysomic for at least four chromosomes selected from the group consisting of human chromosome 6, human chromosome 8, human chromosome 10, and human chromosome Y; or human chromosome 7, human chromosome 16, human chromosome 18, and human chromosome 20,

   wherein the presence of prostate cells that are polysomic for human chromosome 6, human chromosome 8, human chromosome 10, or human chromosome Y; or human chromosome 7, human chromosome 16, human chromosome 18, or human chromosome 20 indicates that the subject has a prostate carcinoma.

2. The method of any one of the preceding claims, wherein (b) or (c), or a combination thereof, is performed at room temperature.

3. The method of any one of the preceding claims, further comprising one or more additional steps selected from the group consisting of fixing the prostate cells on a solid support, pretreating the prostate cells with at least one protease, denaturing the prostate cells, and counterstaining the prostate cells.

4. The method of any one of the preceding claims, wherein the urine sample is voided urine obtained from the subject subsequent to the application of pressure to the subject's prostate gland.

5. The method of any preceding claim, wherein the probes are nucleic acid probes, preferably DNA probes, and more preferably oligonucleotide probes.

6. The method of Claim 5, wherein the oligonucleotide probes have a length of about 20 nucleotides to about 50 nucleotides, and preferably wherein the oligonucleotide probes have a length of about 30 nucleotides.

7. The method of any one of Claims 5-6, wherein the probes are single-stranded.

8. The method of any one of Claims 5-7, wherein the probes are centromeric probes.

9. The method of Claim 1

wherein the at least four chromosome-specific probes include a chromosome-specific probe for human chromosome 6, a chromosome-specific probe for human chromosome 8, a chromosome-specific probe for human chromosome 10, and a chromosome-specific probe for human chromosome Y; and step (e) comprises determining whether the prostate cells include prostate cells that are polysomic for at least four chromosomes selected from the group consisting of chromosome 6, chromosome 8, chromosome 10 and chromosome Y,

wherein the presence of prostate cells that are polysomic for chromosome 6, chromosome 8, chromosome 10 or chromosome Y, indicates that the subject has a prostate carcinoma.

10. A kit for detecting polysomic prostate cancer cells in a sample from a human subject, comprising at least two chromosome-specific probes,
wherein the kit consists of a set of four chromosome-specific probes, consisting of a chromosome-specific probe for human chromosome 6, a chromosome-specific probe for human chromosome 8, a chromosome-specific probe for human chromosome 10, and a chromosome- specific probe for human chromosome Y; or,
a chromosome-specific probe for human chromosome 7, a chromosome-specific probe for human chromosome 16, a chromosome- specific probe for human chromosome 18, and a chromosome-specific probe for human chromosome 20;
wherein the probes are DNA probes, preferably nucleic acid probes; wherein the nucleic acid probes are single stranded, and wherein each chromosome-specific probe has a detectable label and a nucleotide sequence that is complementary to a repetitive genomic sequence of a specific human chromosome; and optionally,
reagents for performing in situ hybridization.

11. The kit of Claim 10, wherein the nucleic acid probes are oligonucleotide probes preferably, wherein the oligonucleotide probes have a length of about 20 to about 50 nucleotides, more preferably about 30 nucleotides.

12. The kit of Claim 10, wherein the nucleic acid probes are prepared from plasmids, bacterial artificial chromosomes (BACs), yeast artificial chromosomes (YACs) or genomic DNA.

13. The kit of any one of Claims 10-12, wherein the nucleic acid probes are centromeric probes.

14. The kit of any one of Claims 10-13, wherein each probe has a different detectable label, or the same detectable label, preferably wherein the detectable label is a fluor.

15. A kit for detecting prostate cancer cells in a sample from a human subject, consisting of a set of probes consisting of four chromosome-specific, single- stranded DNA oligonucleotide probes, wherein the four chromosome- specific probes each has a different detectable label and a nucleotide sequence that is complementary to a repetitive genomic sequence of a specific human chromosome, and is specific for a different human chromosome selected from the group consisting of human chromosome Y, human chromosome 6, human chromosome 7, human chromosome 8, human chromosome 10, human chromosome 16, human chromosome 18 and human chromosome 20; and optionally, additionally consisting of reagents for performing in situ hybridisation.

**Patentansprüche**

1. Verfahren zum Diagnostizieren, ob ein menschliches Subjekt ein Prostatakarzinom aufweist, Folgendes umfassend:

   a) Bereitstellen einer Probe, die Prostatazellen von dem Subjekt enthält;
   b) Hybridisieren eines Satzes von mindestens vier chromosomenspezifischen Sonden an die Prostatazellen, wobei jede chromosomenspezifische Sonde eine erfassbare Markierung und eine Nukleotidsequenz aufweist, die einer sich wiederholenden genomischen Sequenz eines spezifischen menschlichen Chromosoms komplementär ist, und spezifisch für ein anderes menschliches Chromosom ist;
   wobei der Satz chromosomenspezifischer Sonden Folgendes beinhaltet:

   i. eine chromosomenspezifische Sonde für das menschliche Chromosom 6, eine chromosomenspezifische Sonde für das menschliche Chromosom 8, eine chromosomenspezifische Sonde für das menschliche Chromosom 10 und eine chromosomenspezifische Sonde für das menschliche Chromosom Y; oder,
   ii. eine chromosomenspezifische Sonde für das menschliche Chromosom 7, eine chromosomenspezifische Sonde für das menschliche Chromosom 16, eine chromosomenspezifische Sonde für das menschliche Chromosom 18 und eine chromosomenspezifische Sonde für das menschliche Chromosom 20;

   c) Entfernen von nicht-hybridisierten Sonden;
   d) Erfassen der Markierungen auf chromosomenspezifischen Sonden, die an die Prostatazellen hybridisiert haben; und
   e) Bestimmen, ob die Prostatazellen Prostatazellen beinhalten, die für mindestens vier Chromosomen polysom sind, ausgewählt aus der Gruppe bestehend aus dem menschlichen Chromosom 6, dem menschlichen Chromosom 8, dem menschlichen Chromosom 10 und dem menschlichen Chromosom Y, oder dem menschlichen Chromosom 7, dem menschlichen Chromosom 16, dem menschlichen Chromosom 18 und dem menschlichen Chromosom 20,
   wobei die Anwesenheit von Prostatazellen, die für das menschliche Chromosom 6, das menschliche Chromosom 8, das menschliche Chromosom 10 oder das menschliche Chromosom Y, oder das menschliche Chromosom 7, das menschliche Chromosom 16, das menschliche Chromosom 18 oder das menschliche Chromosom 20 polysom sind, anzeigt, dass das Subjekt ein Prostatakarzinom aufweist.

2. Verfahren nach einem der vorhergehenden Ansprüche, wobei (b) oder (c) oder eine Kombination davon bei Raumtemperatur durchgeführt wird.

3. Verfahren nach einem der vorhergehenden Ansprüche, ferner umfassend einen oder mehrere zusätzliche Schritte, ausgewählt aus der Gruppe bestehend aus Befestigen der Prostatazellen auf einem festen Träger, Vorbehandeln die Prostatazellen mit mindestens einer Protease, Denaturieren der Prostatazellen, und Gegenfärben der Prostatazellen.

4. Verfahren nach einem der vorhergehenden Ansprüche, wobei es sich bei der Urinprobe um ausgeschiedenen Urin handelt, der von dem Subjekt nach dem Ausüben von Druck auf die Prostata des Subjekts erhalten wird.

5. Verfahren nach einem der vorhergehenden Ansprüche, wobei die Sonden Nukleinsäuresonden, bevorzugt DNA-Sonden und stärker bevorzugt Oligonukleotidsonden sind.

6. Verfahren nach Anspruch 5, wobei die Oligonukleotidsonden eine Länge von etwa 20 Nukleotiden bis etwa 50 Nukleotiden aufweisen, und vorzugsweise wobei die Oligonukleotidsonden eine Länge von ungefähr 30 Nukleotiden aufweisen.

7. Verfahren nach einem der Ansprüche 5-6, wobei: die Sonden einzelsträngig sind.

8. Verfahren nach einem der Ansprüche 5-7, wobei die Sonden zentromere Sonden sind.

9. Verfahren nach Anspruch 1, wobei die mindestens vier chromosomenspezifischen Sonden Folgendes beinhalten: eine chromosomenspezifische Sonde für das menschliche Chromosom 6, eine chromosomenspezifische Sonde für das menschliche Chromosom 8, eine chromosomenspezifische Sonde für das menschliche Chromosom 10 und eine chromosomenspezifische Sonde für das menschliche Chromosom Y; und Schritt (e) das Bestimmen umfasst, ob die Prostatazellen Prostatazellen beinhalten, die für mindestens vier Chromosomen polysom sind, ausgewählt aus der Gruppe bestehend aus dem Chromosom 6, dem Chromosom 8, dem Chromosom 10 und dem Chromosom Y, wobei das Vorhandensein von Prostatazellen, die für das Chromosom 6, das Chromosom 8, das Chromosom 10 oder das Chromosom Y polysom sind, anzeigt, dass das Subjekt ein Prostatakarzinom aufweist.

10. Kit zum Erfassen von polysomen Prostatakrebszellen in einer Probe von einem menschlichen Subjekt, umfassend mindestens zwei chromosomenspezifische Sonden, wobei das Kit aus einem Satz von vier chromosomenspezifischen Sonden besteht, bestehend aus einer chromosomenspezifischen Sonde für das menschliche Chromosom 6, einer chromosomenspezifischen Sonde für das menschliche Chromosom 8, einer chromosomenspezifischen Sonde für das menschliche Chromosom 10 und einer chromosomenspezifischen Sonde für das menschliche Chromosom Y, oder

einer chromosomenspezifischen Sonde für das menschliche Chromosom 7, einer chromosomenspezifischen Sonde für das menschliche Chromosom 16, einer chromosomenspezifischen Sonde für das menschliche Chromosom 18 und einer chromosomenspezifischen Sonde für das menschliche Chromosom 20;

wobei die Sonden DNA-Sonden, vorzugsweise Nukleinsäuresonden sind;

wobei die Nukleinsäuresonden einzelsträngig sind und wobei jede chromosomenspezifische Sonde eine erfassbare Markierung und eine Nukleotidsequenz aufweist, die zu einer sich wiederholenden genomischen Sequenz eines spezifischen menschlichen Chromosoms komplementär ist; und

optional Reagenzien zum Durchführen einer *in-situ*-Hybridisierung.

11. Kit nach Anspruch 10, wobei die Nukleinsäuresonden Oligonukleotidsonden sind, vorzugsweise wobei die Oligonukleotidsonden eine Länge von etwa 20 bis etwa 50 Nukleotiden, stärker bevorzugt etwa 30 Nukleotiden aufweisen.

12. Kit nach Anspruch 10, wobei die Nukleinsäuresonden aus Plasmiden, künstlichen Bakterienchromosomen (*bacterial artificial chromosomes* - BACs), künstlichen Hefechromosomen (*yeast artificial chromosomes* - YACs) oder genomischer DNA hergestellt werden.

13. Kit nach einem der Ansprüche 10-12, wobei die Nukleinsäuresonden zentromere Sonden sind.

14. Kit nach einem der Ansprüche 10-13, wobei jede Sonde eine andere erfassbare Markierung oder dieselbe erfassbare Markierung aufweist, vorzugsweise wobei die erfassbare Markierung ein Fluor ist.

15. Kit zum Erfassen von Prostatakrebszellen in einer Probe von einem menschlichen Subjekt, bestehend aus einem Satz von Sonden, bestehend aus vier chromosomenspezifische, einzelsträngige DNA-Oligonukleotidsonden, wobei die vier chromosomenspezifischen Sonden jeweils Folgendes aufweisen: eine unterschiedliche erfassbare Markierung und eine Nukleotidsequenz, die zu einer sich wiederholenden genomischen Sequenz eines spezifischen menschlichen Chromosoms komplementär ist und für ein anderes menschliches Chromosom spezifisch ist, das aus der Gruppe ausgewählt ist bestehend aus dem menschlichen Chromosom Y, dem menschlichen Chromosom 6, dem menschlichen Chromosom 7, dem menschlichen Chromosom 8, dem menschlichen Chromosom 10, dem menschlichen Chromosom 16, dem menschlichen Chromosom 18 und dem menschlichen Chromosom 20; und optional zusätzlich bestehend aus Reagenzien zum Durchführen einer *in-situ*-Hybridisierung.

## Revendications

1. Procédé permettant de diagnostiquer si un sujet humain a un carcinome de la prostate, comprenant :

    a) l'utilisation d'un échantillon contenant des cellules de la prostate du sujet ;
    b) l'hybridation d'un ensemble d'au moins quatre sondes propres aux chromosomes aux cellules de la prostate, chaque sonde propre aux chromosomes possédant un marqueur détectable et une séquence nucléotidique qui est complémentaire à une séquence génomique répétitive d'un chromosome humain spécifique, et étant spécifique pour un chromosome humain différent ;
    dans lequel l'ensemble de sondes propres aux chromosomes comprend

        i. une sonde propre aux chromosomes pour le chromosome 6 humain, une sonde propre aux chromosomes pour le chromosome 8 humain, une sonde propre aux chromosomes pour le chromosome 10 humain et une sonde propre aux chromosomes pour le chromosome Y humain ; ou
        ii. une sonde propre aux chromosomes pour le chromosome 7 humain, une sonde propre aux chromosomes pour le chromosome 16 humain, une sonde propre aux chromosomes pour le chromosome 18 humain et une sonde propre aux chromosomes pour le chromosome 20 humain ;

c) le retrait des sondes non hybridées ;

d) la détection des marqueurs sur les sondes propres aux chromosomes qui se sont hybridées aux cellules de la prostate ; et

e) la détermination du fait que les cellules de la prostate comprennent des cellules de la prostate qui sont polysomiques pour au moins quatre chromosomes choisis dans le groupe constitué par le chromosome 6 humain, le chromosome 8 humain, le chromosome 10 humain et le chromosome Y humain ; ou

par le chromosome 7 humain, le chromosome 16 humain, le chromosome 18 humain et le chromosome 20 humain,

la présence de cellules de la prostate qui sont polysomiques pour le chromosome 6 humain, pour le chromosome 8 humain, pour le chromosome 10 humain ou pour le chromosome Y humain ; ou

pour le chromosome 7 humain, pour le chromosome 16 humain, pour le chromosome 18 humain ou pour le chromosome 20 humain indique que le sujet a un carcinome de la prostate.

2. Procédé selon l'une quelconque des revendications précédentes, dans lequel l'étape (b) ou (c), ou une combinaison de celles-ci, a lieu à température ambiante.

3. Procédé selon l'une quelconque des revendications précédentes, comprenant en outre au moins une étape supplémentaire choisie dans le groupe consistant à fixer les cellules de la prostate sur un support solide, à prétraiter les cellules de la prostate avec au moins une protéase, à dénaturer les cellules de la prostate et à colorer les cellules de la prostate.

4. Procédé selon l'une quelconque des revendications précédentes, dans lequel l'échantillon d'urine est de l'urine excrétée obtenue du sujet après l'application d'une pression sur la glande prostatique du sujet.

5. Procédé selon l'une des revendications précédentes, dans lequel les sondes sont des sondes d'acides nucléiques, de préférence des sondes ADN, et plus préférablement des sondes oligonucléotidiques.

6. Procédé selon la revendication 5, dans lequel les sondes oligonucléotidiques ont une longueur d'environ 20 nucléotides à environ 50 nucléotides, et de préférence dans lequel les sondes oligonucléotidiques ont une longueur d'environ 30 nucléotides.

7. Procédé selon l'une quelconque des revendications 5 à 6, dans lequel les sondes sont monocaténaires.

8. Procédé selon l'une quelconque des revendications 5 à 7, dans lequel les sondes sont des sondes centromériques.

9. Procédé selon la revendication 1, dans lequel les au moins quatre sondes spécifiques des chromosomes comprennent une sonde propre aux chromosomes pour le chromosome 6 humain, une sonde propre aux chromosomes pour le chromosome 8 humain, une sonde propre aux chromosomes pour le chromosome 10 humain et une sonde propre aux chromosomes pour le chromosome Y humain ; et

l'étape (e) comprend la détermination du fait que les cellules de la prostate comprennent des cellules de la prostate qui sont polysomiques pour au moins quatre chromosomes choisis dans le groupe constitué par le chromosome 6, le chromosome 8, le chromosome 10 et le chromosome Y, la présence de cellules de la prostate qui sont polysomiques pour le chromosome 6, pour le chromosome 8, pour le chromosome 10 ou pour le chromosome Y indiquant que le sujet a un cancer de la prostate.

10. Kit de détection de cellules cancéreuses polysomiques de la prostate dans un échantillon provenant d'un sujet humain, comprenant au moins deux sondes spécifiques des chromosomes, le kit comprenant un ensemble de quatre sondes spécifiques des chromosomes, comprenant une sonde propre aux chromosomes pour le chromosome 6 humain, une sonde propre aux chromosomes pour le chromosome 8 humain, une sonde propre aux chromosomes pour le chromosome 10 humain et une sonde propre aux chromosomes pour le chromosome Y humain ; ou

une sonde propre aux chromosomes pour le chromosome 7 humain, une sonde propre aux chromosomes pour le chromosome 16 humain, une sonde propre aux chromosomes pour le chromosome 18 humain et une sonde propre aux chromosomes pour le chromosome 20 humain ;

les sondes étant des sondes ADN, de préférence des sondes d'acides nucléiques ;

28

les sondes d'acides nucléiques étant monocaténaires, et chaque sonde spécifique des chromosomes ayant un marqueur détectable et une séquence nucléotidique qui est complémentaire à une séquence génomique répétitive d'un chromosome humain spécifique ; et

facultativement, des réactifs permettant d'effectuer l'hybridation in situ.

11. Kit selon la revendication 10, dans lequel les sondes d'acides nucléiques sont des sondes oligonucléotidiques de préférence, les sondes oligonucléotidiques ayant une longueur d'environ 20 à environ 50 nucléotides, plus préférablement d'environ 30 nucléotides.

12. Kit selon la revendication 10, dans lequel les sondes d'acides nucléiques sont préparées à partir de plasmides, de chromosomes artificiels bactériens (CAB), de chromosomes artificiels de levure (CAL) ou d'ADN génomique.

13. Kit selon l'une quelconque des revendications 10 à 12, dans lequel les sondes d'acides nucléiques sont des sondes centromériques.

14. Kit selon l'une quelconque des revendications 10 à 13, dans lequel chaque sonde comprend un marqueur détectable différent ou le même marqueur détectable, le marqueur détectable étant de préférence du fluor.

15. Kit de détection de cellules cancéreuses de la prostate dans un échantillon d'un sujet humain, comprenant un ensemble de sondes comprenant quatre sondes oligonucléotidiques d'ADN monocaténaire propres aux chromosomes, les quatre sondes propres aux chromosomes ayant chacune un marqueur détectable différent et une séquence nucléotidique qui est complémentaire à une séquence génomique répétitive d'un chromosome humain spécifique et qui est spécifique pour un chromosome humain différent, choisi dans le groupe constitué par le chromosome Y humain, le chromosome 6 humain, le chromosome 7 humain, le chromosome 8 humain, le chromosome 10 humain, le chromosome 16 humain, le chromosome 18 humain et le chromosome 20 humain ; et facultativement, comprenant en outre des réactifs permettant d'effectuer l'hybridation in situ.

FIG. 1A

FIG. 1B

FIG. 1C

FIG. 1D

FIG. 2

FIG. 3

**Benign vs Cancer**

FIG. 4

**Benign vs Cancer 1st**

0.8841

FIG. 5

**low vs high**

FIG. 6

**Low vs High 1st**

0.6065

FIG 7

FIG. 8

FIG. 9

FIG. 10

FIG. 11

FIG. 12

FIG. 13

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5082830 A
- US 5580990 A
- US 5714327 A
- US 6825330 B
- WO 9201699 A
- WO 9635696 A
- WO 9815546 A
- US 20130203055 A1
- US 20130149705 A1
- US 5719024 A
- US 20130149705 A

**Non-patent literature cited in the description**

- Cancer Facts & Figures. American Cancer Society, 2013, 19-20
- **KONIG et al.** *Human Pathology,* 1996, 720-727
- **BERGERHEIM et al.** *Genes Chromosomes and Cancer,* 1991, 215-220
- **ANN et al.** *BMC Genomics,* 2004, 27
- **NIELSEN et al.** *Science,* 1991, vol. 254, 1497-1500
- **NIELSEN et al.** *J. Biomol. Struct. Dyn.,* 1999, vol. 17, 175-91
- **ALLSHIRE et al.** *Nucleic Acids Res,* 1989, vol. 17 (12), 4611-27
- **CHO et al.** *Nucleic Acids Res,* 1991, vol. 19 (6), 1179-82
- **FOWLER et al.** *Nucleic Acids Res,* 1987, vol. 15 (9), 3929
- **HAAF et al.** *Cell,* 1992, vol. 70 (4), 681-96
- **LEE et al.** *Chromosoma,* 2000, vol. 109 (6), 381-9
- **MAEDA ; SMITHIES.** *Annu Rev Genet,* 1986, vol. 20, 81-108
- **MEYNE ; GOODWIN.** *Chromosoma,* 1994, vol. 103 (2), 99-103
- Localized highly repetitive DNA sequences in vertebrate genomes. **MIKLOS.** Molecular evolutionary genetics. Plenum Publishing Corp, 1985, 241-321
- **TAGARRO et al.** *Hum Genet,* 1994, vol. 93 (2), 125-8
- **WAYE ; WILLARD.** *PNAS USA,* 1989, vol. 86 (16), 6250-4
- **WILLARD ; WAYE.** *J Mol Evol,* 1987, vol. 25 (3), 207-14
- **VISSEL ; CHOO.** *Nucleic Acids Res.,* 1987, vol. 15 (16), 6751-6752
- **GLICK ; PASTERNAK.** Molecular Biotechnology: Principles and Applications of Recombinant DNA. ASM Press, 1998
- **BRUCHEZ et al.** *Science,* 1998, vol. 281, 2013-2016
- **WARREN ; NIE.** *Science,* 1998, vol. 281, 2016-2018
- **HERNANDEZ-SANTOSET et al.** *Anal. Chem.,* 2005, vol. 77, 2868-2874
- **RAAP et al.** *BioTechniques,* 2004, vol. 37, 1-6
- **HEETEBRIJ et al.** *ChemBioChem,* 2003, vol. 4, 573-583
- **VAN DE RIJKE et al.** *Analytical Biochemistry,* 2003, vol. 321, 71-78
- **GUPTA et al.** *Nucleic Acids Research,* 2003, vol. 31, e13
- **VAN GIJLSWIJK et al.** *Clinical Chemistry,* 2002, vol. 48, 1352-1359
- **ALERS et al.** *Genes, Chromosomes & Cancer,* 1999, vol. 25, 301-305
- **WIEGANT et al.** *Cytogenetics and Cell Genetics,* 1999, vol. 87, 7-52
- **JELSMA et al.** *Journal of NIH Research,* 1994, vol. 5, 82
- **VAN BELKUM et al.** *BioTechniques,* 1994, vol. 16, 148-153
- **VAN BELKUM et al.** *Journal of Virological Methods,* 1993, vol. 45, 189-200
- **HERMAN.** Bioconjugate Chemistry. Academic Press, 1996
- Hybridization with Nucleic Acid Probes. **TIJSSEN.** Laboratory Techniques in Biochemistry and Molecular Biology. Elsevier, 1993, vol. 24
- **SCHROECK et al.** *Science,* 1996, vol. 273, 494-497
- **SPEICHER et al.** *Nature Genetics,* 1996, vol. 12, 368-375
- **GALL ; PARDUE.** *Methods in Enzymology,* 1981, vol. 21, 470-480
- **HENDERSON.** *International Review of Cytology,* 1982, vol. 76, 1-46

- **ANGERER et al.** Genetic Engineering: Principles and Methods. Plenum Press, 1985
- **MEHES et al.** *Cytometry,* 2000, vol. 42, 357-362
- **TRASK et al.** *Science,* 1985, vol. 230, 1401-1402